(19) Europäisches Patentamt
European Patent Office
Office européen des brevets

(11) **EP 4 270 311 B1**

(12) **EUROPEAN PATENT SPECIFICATION**

(45) Date of publication and mention
of the grant of the patent:
**24.12.2025 Bulletin 2025/52**

(21) Application number: **23150023.2**

(22) Date of filing: **02.01.2023**

(51) International Patent Classification (IPC):
*G06T 7/00* *(2017.01)*

(52) Cooperative Patent Classification (CPC):
**G06T 7/0012;** G06T 2207/10072;
G06T 2207/20081; G06T 2207/30056;
G06T 2207/30096; G06T 2207/30101

(54) **IMAGE IDENTIFICATION PROGRAM, IMAGE IDENTIFICATION METHOD, IMAGE IDENTIFICATION DEVICE**

BILDIDENTIFIZIERUNGSPROGRAMM, BILDIDENTIFIZIERUNGSVERFAHREN, BILDIDENTIFIZIERUNGSVORRICHTUNG

PROGRAMME D'IDENTIFICATION D'IMAGE, PROCÉDÉ D'IDENTIFICATION D'IMAGE, DISPOSITIF D'IDENTIFICATION D'IMAGE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **25.04.2022 JP 2022071272**

(43) Date of publication of application:
**01.11.2023 Bulletin 2023/44**

(73) Proprietor: **FUJITSU LIMITED**
**Kawasaki-shi, Kanagawa 211-8588 (JP)**

(72) Inventors:
• **Karasudani, Ayu**
**Kawasaki-shi, Kanagawa 211-8588 (JP)**
• **Ishihara, Masaki**
**Kawasaki-shi, Kanagawa 211-8588 (JP)**

(74) Representative: **Hoffmann Eitle**
**Patent- und Rechtsanwälte PartmbB**
**Arabellastraße 30**
**81925 München (DE)**

(56) References cited:
**US-A1- 2020 380 675**

• FANTAZZINI ALICE ET AL: "3D Automatic Segmentation of Aortic Computed Tomography Angiography Combining Multi-View 2D Convolutional Neural Networks", CARDIOVASCULAR ENGINEERING AND TECHNOLOGY, SPRINGER INTERNATIONAL PUBLISHING, CHAM, vol. 11, no. 5, 11 August 2020 (2020-08-11), pages 576 - 586, XP037253898, ISSN: 1869-408X, [retrieved on 20200811], DOI: 10.1007/S13239-020-00481-Z

• SUNYI ZHENG ET AL: "Efficient convolutional neural networks for multi-planar lung nodule detection: improvement on small nodule identification", ARXIV.ORG, CORNELL UNIVERSITY LIBRARY, 201 OLIN LIBRARY CORNELL UNIVERSITY ITHACA, NY 14853, 13 January 2020 (2020-01-13), XP081705199

**Description**

FIELD

The embodiments discussed herein are related to an image identification program, an image identification method, and an image identification device.

BACKGROUND

Medical images obtained by computed tomography (CT), magnetic resonance imaging (MRI), and the like are widely used for diagnosis of various diseases. A doctor needs to interpret a large number of images in diagnostic imaging using medical images, which places a heavy burden on the doctor. Therefore, there has been a demand for a technique of supporting diagnostic work of a doctor in some way using a computer.

The following proposals have been made as techniques for supporting diagnosis using medical images. For example, there has been proposed a computing system that uses multiple individual neural networks to process each of multiple two-dimensional images of biological tissue structures. Furthermore, there has been proposed a segmentation method that converts a three-dimensional volume including a pericardium into three sets of two-dimensional images and employs multiple independently trained neural networks, each of which processes one of the three sets.

U.S. Patent No. 9968257 and U.S. Patent Application Publication No. 2020/0320751 are disclosed as related art.

Further background is provided in the following document.

US 2020/0380675 A1 discloses an automated end-to-end pipeline for lesion detection and segmentation.

SUMMARY

There is provided an image identification program as set out in Claim 1. There is also provided an image identification device as set out in Claim 3. There is also provided an image identification method as set out in Claim 4.

BRIEF DESCRIPTION OF DRAWINGS

FIG. 1 is a diagram illustrating an exemplary configuration and exemplary processing of an image identification device according to a first embodiment;

FIG. 2 is a diagram illustrating an exemplary configuration of a diagnostic support processing system according to a second embodiment;

FIG. 3 is a diagram illustrating an exemplary hardware configuration of the image identification device;

FIG. 4 is a first diagram illustrating a comparative example of a lesion identification process;

FIG. 5 is a second diagram illustrating a comparative example of the lesion identification process;

FIG. 6 is a third diagram illustrating a comparative example of the lesion identification process;

FIG. 7 is a diagram for explaining a process of generating identification input data according to the second embodiment;

FIG. 8 is a diagram illustrating an exemplary configuration of processing functions of a training processing device and the image identification device;

FIG. 9 is a diagram illustrating an exemplary internal configuration of a training data generation unit;

FIG. 10 is a diagram for explaining a process of annotation and distance image generation;

FIG. 11 is a diagram illustrating a first example of a distance image generation method;

FIG. 12 is a diagram illustrating a second example of the distance image generation method;

FIG. 13 is a diagram illustrating a processing procedure of normal 3D patch determination;

FIG. 14 is a diagram illustrating a processing procedure of tumor 3D patch determination;

FIG. 15 is a diagram for explaining training data input to a classifier training unit;

FIG. 16 is an exemplary flowchart illustrating a processing procedure of an entire training process;

FIG. 17 is an exemplary flowchart (part 1) of a procedure of a combined 2D patch generation process;

FIG. 18 is an exemplary flowchart (part 2) of a procedure of the combined 2D patch generation process;

FIG. 19 is an exemplary flowchart (part 3) of a procedure of the combined 2D patch generation process;

FIG. 20 is a diagram illustrating an exemplary internal configuration of an input data generation unit; and

FIG. 21 is an exemplary flowchart illustrating an identification processing procedure of the image identification device.

DESCRIPTION OF EMBODIMENTS

[TECHNICAL PROBLEM]

Incidentally, in an identification process for identifying, from a medical image, which of a plurality of lesion-related states an imaged region is in, it may be difficult to distinguish between two specific states depending on the disease. For example, there is a case where a specific lesion area and a normal specific part in a body appear with similar brightness in an image while three-dimensional shapes of the former lesion area and the latter specific part are different. In this case, it is highly likely that both of them may be distinguished from each other if the lesion area and the specific part appear in different shapes in the image. However, both of them may appear in the image in similar shapes in some cases, and in that case, both of them may not be accurately distinguished from each other.

As an example, a tumor and a blood vessel appear with similar brightness in an MR image of a hepatobiliary phase obtained by imaging a liver using a linear MRI hepatographic imaging agent (gadoxetate sodium). A three-dimensional shape of tumor is spherical while a three-dimensional shape of the blood vessel is an elongated tubular shape. In this case, while the tumor appears in the MR image in a circular shape or an oval shape, the blood vessel may also appear in the MR image in a circular shape or an oval shape, and in this case, the blood vessel region may be erroneously identified as the tumor region.

In one aspect, the embodiments aim to provide an image identification program, an image identification method, an image identification device, and an information processing system capable of highly accurately identifying a lesion-related state from a medical image.

[SOLUTION TO PROBLEM]

According to an aspect of the embodiments, an image identification program that causes at least one computer to execute a process, the process includes acquiring a three-dimensional partial area of a certain size from three-dimensional volume data generated based on a plurality of tomographic images obtained by imaging an inside of a human body; generating a plurality of projection images by performing extreme intensity projection on each voxel value of the partial area in a plurality of mutually orthogonal directions, the extreme intensity projection being one intensity projection selected from minimum intensity projection and maximum intensity projection; and specifying one state of the partial area among a plurality of states based on the plurality of projection images.

[ADVANTAGEOUS EFFECTS OF INVENTION]

In one aspect, a lesion-related state may be highly accurately identified from a medical image.

Hereinafter, embodiments will be described with reference to the drawings.

[First Embodiment]

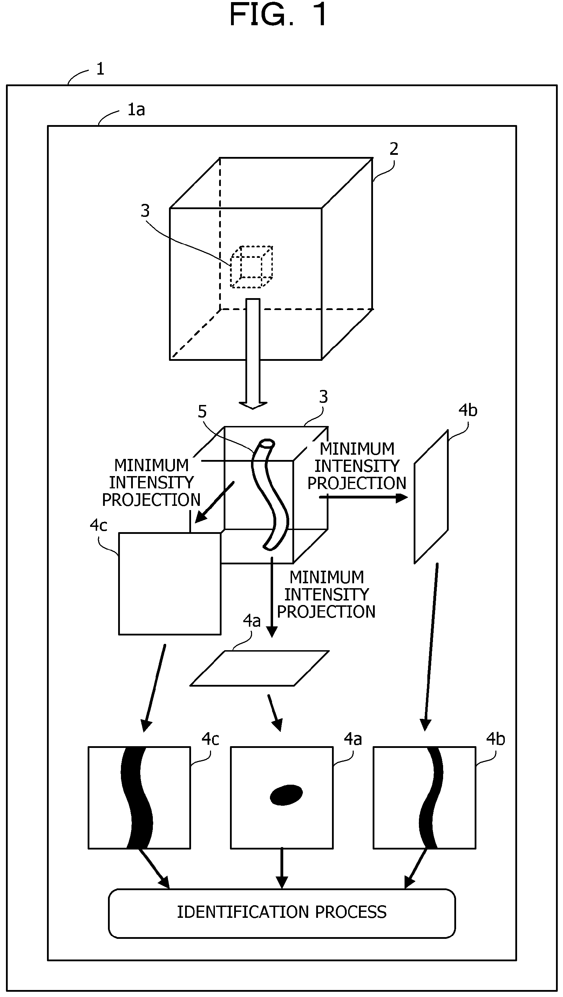

FIG. 1 is a diagram illustrating an exemplary configuration and exemplary processing of an image identification device according to a first embodiment. An image identification device 1 illustrated in FIG. 1 is an information processing device that obtains multiple tomographic images obtained by imaging the inside of a human body and identifies, based on those tomographic images, which of a plurality of lesion-related states the imaged region is in. For example, in a case where a tomographic image of a region including a liver is captured, a tumor region or a normal region is identified as the plurality of states. Note that a tomographic image is, for example, a medical image such as a CT image, MR image, or the like.

The image identification device 1 includes a processing unit 1a. The processing unit 1a is, for example, a processor. The processing unit 1a performs a process as follows.

The processing unit 1a generates three-dimensional volume data 2 based on the multiple tomographic images described above. The processing unit 1a cuts out a three-dimensional partial area 3 of a predetermined size from the generated volume data 2. This partial area 3 serves as a unit for lesion identification.

The processing unit 1a carries out minimum intensity projection or maximum intensity projection of each voxel value (e.g., luminance value) of the partial area 3 in a plurality of mutually orthogonal directions, thereby generating a plurality of projection images. In the example of FIG. 1, the minimum intensity projection is carried out in three directions to generate projection images 4a to 4c. The processing unit 1a identifies which of the plurality of states described above the partial area 3 is in based on the generated projection images 4a to 4c. As a result, it becomes possible to identify a lesion-related state highly accurately.

Here, as an example, it is assumed that a first state and a second state are distinguished as lesion states. In this case, which of the minimum intensity projection or the maximum intensity projection is to be adopted may be determined from a condition of which of the first state and the second state appears relatively brighter in the volume data 2 and a condition of in what shape each region of the first state and the second state may be present in the volume data 2, for example.

In the following descriptions, it is assumed that the first state appears darker and the projection images 4a to 4c are generated by the minimum intensity projection. Furthermore, it is assumed that a region in the first state is normally spherical in the volume data 2.

Meanwhile, it is assumed that an area that appears with brightness similar to that of the first state may be generated in a region in the second state as well. For example, it is assumed that a specific part 5, which is identified as being in the second state, may appear in the volume data 2 with brightness similar to that of the first state. In the example of FIG.1, it is assumed that such a specific part 5 has an elongated tubular shape in the volume data 2. Note that a blood vessel is assumed as such a specific part 5, for example.

For example, a case will be considered in which the first state and the second state described above are distinguished based on the tomographic image from which the volume data 2 is generated or a tomographic image in any direction generated from the volume data 2. Since the region in the first state is spherical, this region appears in a circular shape or in an oval shape in the tomographic image. Meanwhile, the specific part 5 in the elongated tubular shape may appear long and thin, or may appear in a circular shape or in an oval shape in the tomographic image. For example, since the specific part 5 may appear in the tomographic image with a shape and brightness similar to those of the region of the first state, the region of the specific part 5 may be erroneously identified as the first region according to the identification method described above.

In order to address such a problem, in the present embodiment, the projection images 4a to 4c obtained by the minimum intensity projection in the plurality of mutually orthogonal directions are used to suppress the occurrence of erroneous identification. For example, the region of the first state appears in a circular shape or in an oval shape in any of the projection images 4a to 4c. On the other hand, the specific part 5 appears in an elongated shape in at least one of the projection images 4a to 4c. In the example of FIG. 1, while the specific part 5 appears in an oval shape in the projection image 4a, it appears in an elongated shape in the projection images 4b and 4c. Therefore, by executing the identification process based on the projection images 4a to 4c, it becomes possible to suppress the possibility of erroneously identifying the region of the specific part 5 as the first region and to improve the identification accuracy.

Note that, in the example of FIG. 1, the projection images 4a to 4c in three directions are generated based on the partial area 3, and are used for the identification process. However, for example, projection images in two mutually orthogonal directions may be generated, and each of the projection images may be used for the identification process. Even in this case, for example, it is highly likely that the specific part 5 described above may appear in an elongated shape in at least one projection image. Therefore, even in this case, the identification accuracy may improve.

[Second Embodiment]

Next, a system capable of distinguishing, as a lesion-related condition, between a tumor region and a normal region in a liver will be described.

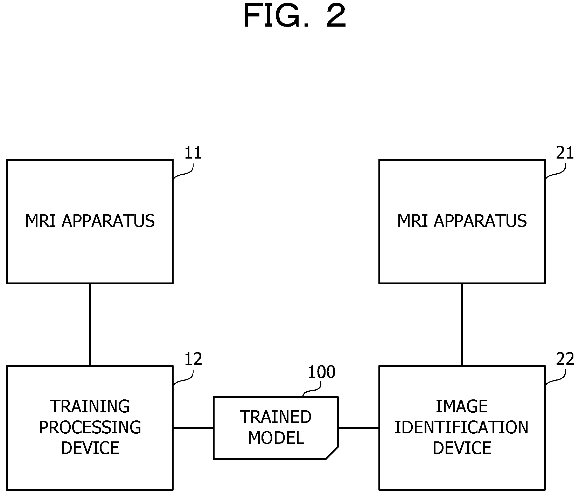

FIG. 2 is a diagram illustrating an exemplary configuration of a diagnostic support processing system according to a second embodiment. The diagnostic support system illustrated in FIG. 2 is a system for supporting diagnostic imaging based on MRI imaging, and includes MRI apparatuses 11 and 21, a training processing device 12, and an image identification device 22. Note that the image identification device 22 is an example of the image identification device 1 illustrated in FIG. 1.

The MRI apparatuses 11 and 21 captures MR images of a human body. In the present embodiment, the MRI apparatuses 11 and 21 captures a predetermined number of tomographic images of axial planes in an abdominal region including a liver while changing a position in the height direction (direction perpendicular to the axial planes) of the human body at predetermined intervals. Furthermore, in the present embodiment, it is assumed that the MRI apparatuses 11 and 21 performs imaging using a linear MRI hepatographic imaging agent (gadoxetate sodium).

The image identification device 22 generates three-dimensional volume data from each tomographic image captured by the MRI apparatus 21, and cuts out a three-dimension (3D) patch, which is a three-dimensional area of a certain size, from the volume data. The image identification device 22 performs a lesion identification process on each 3D patch using a classifier based on a trained model 100 generated by the training processing device 12. This lesion identification process identifies whether the 3D patch is a tumor region or a normal region. Furthermore, in this lesion identification process, the image identification device 22 generates a 2D image ("combined 2D patch" to be described later) as input data for identification based on the 3D patch, and inputs the 2D image to the classifier, thereby performing the lesion identification process.

The training processing device 12 generates, by machine learning, a weight of the trained model 100 to be used in the lesion identification process by the image identification device 22. For this model generation process, the training processing device 12 generates training data from each tomographic image captured by the MRI apparatus 11, and carries out machine learning using the generated training data. The weight of the trained model 100 generated by the training processing device 12 is read into the image identification device 22 via, for example, a network or a portable recording medium.

Note that captured images may be input to the training processing device 12 and to the image identification device 22 from the same MRI apparatus 11. Furthermore, the training processing device 12 may obtain captured images from the MRI apparatus via a recording medium or the like instead of directly obtaining them. Moreover, the training processing device 12 and the image identification device 22 may be the same information processing device.

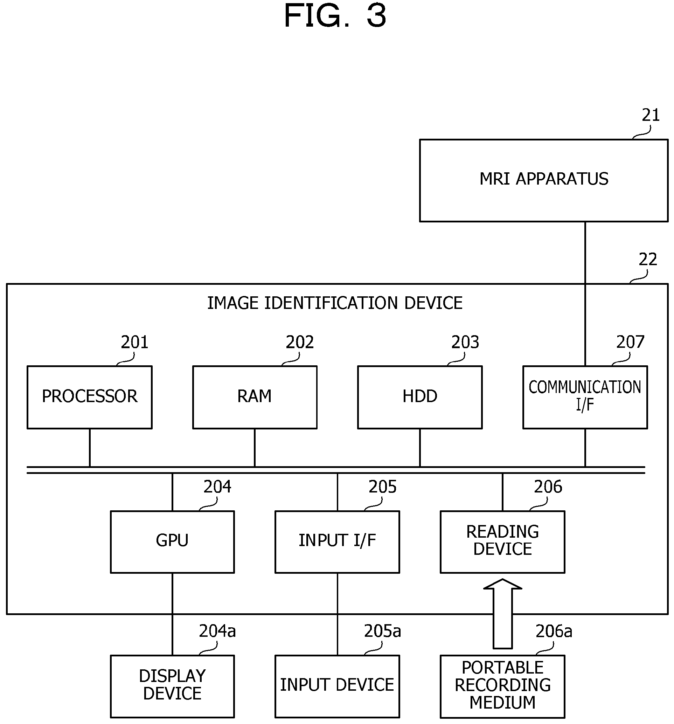

FIG. 3 is a diagram illustrating an exemplary hardware configuration of the image identification device. The image identification device 22 is implemented as a computer having a hardware configuration as illustrated in FIG. 3, for example. As illustrated in FIG. 3, the image identification device 22 includes a processor 201, a random access memory (RAM) 202, a hard disk drive (HDD) 203, a graphics processing unit (GPU) 204, an input interface (I/F) 205, a reading device 206, and a communication interface (I/F) 207.

The processor 201 integrally controls the entire image identification device 22. The processor 201 is, for example, a central processing unit (CPU), a micro processing unit (MPU), a digital signal processor (DSP), an application specific integrated circuit (ASIC), or a programmable logic device (PLD). Furthermore, the processor 201 may be a combination of two or more elements of a CPU, an MPU, a DSP, an ASIC, and a PLD. Note that the processor 201 is an example of the processing unit 1a illustrated in FIG. 1.

The RAM 202 is used as a main storage device of the image identification device 22. The RAM 202 temporarily stores at least a part of operating system (OS) programs and application programs to be executed by the processor 201. Furthermore, the RAM 202 stores various kinds of data needed for processing to be performed by the processor 201.

The HDD 203 is used as an auxiliary storage device of the image identification device 22. The HDD 203 stores the OS programs, the application programs, and various kinds of data. Note that another type of nonvolatile storage device, such as a solid state drive (SSD) or the like, may be used as the auxiliary storage device.

A display device 204a is coupled to the GPU 204. The GPU 204 displays an image on the display device 204a in accordance with an instruction from the processor 201. Examples of the display device 204a include a liquid crystal display, an organic electroluminescence (EL) display, and the like.

An input device 205a is coupled to the input interface 205. The input interface 205 transmits a signal output from the input device 205a to the processor 201. Examples of the input device 205a include a keyboard, a pointing device, and the like. Examples of the pointing device include a mouse, a touch panel, a tablet, a touch pad, a track ball, and the like.

A portable recording medium 206a is attached to and detached from the reading device 206. The reading device 206 reads data recorded in the portable recording medium 206a, and transmits it to the processor 201. Examples of the portable recording medium 206a include an optical disk, a semiconductor memory, and the like.

The communication interface 207 exchanges data with another device, such as the MRI apparatus 21, via a network.

Processing functions of the image identification device 22 may be implemented by the hardware configuration as described above. Note that the training processing device 12 may also be implemented as a computer having a hardware configuration as illustrated in FIG. 3.

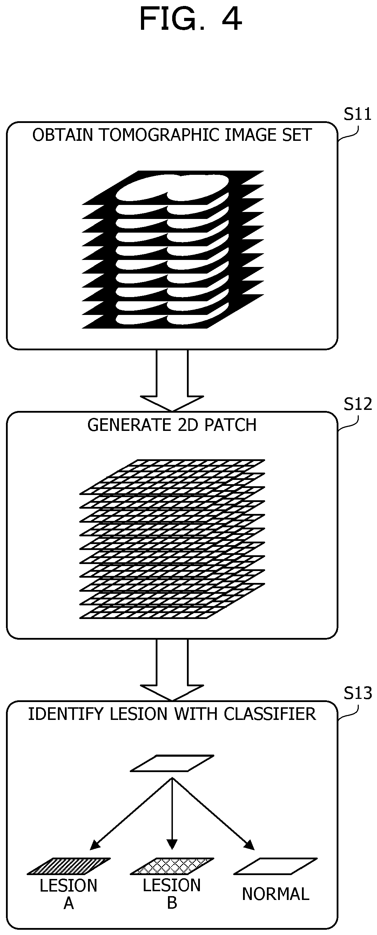

Next, a comparative example of the lesion identification process will be described with reference to FIGs. 4 to 6.

FIG. 4 is a first diagram illustrating a comparative example of the lesion identification process. There is a method using a classifier generated by machine learning as a lesion identification method. For example, as illustrated in FIG. 4, there is a method of cutting out a "patch", which is an image area of a certain size, from each tomographic image and performing the lesion identification process in patch units. Note that, in the following descriptions, a patch cut out from a tomographic image will be referred to as a "2D patch" to distinguish it from a three-dimensional patch (3D patch) to be described later.

In the example of FIG. 4, a tomographic image set is obtained from the MRI apparatus (step S11), and a 2D patch is generated from each tomographic image included in the tomographic image set (step S12). A 2D patch is generated by, for example, a tomographic image being divided into a fixed size such as 16 pixels × 16 pixels. Alternatively, the 2D patch may be generated by the tomographic image being cut out with a window of the same size as the 2D patch shifting on the tomographic image. For example, a window of 16 pixels × 16 pixels is shifted in the horizontal direction by 8 pixels, thereby cutting out a 2D patch of 16 pixels × 16 pixels.

The 2D patch generated in this manner is input to a classifier based on a trained model generated by prior machine learning. As a result, lesions in the 2D patches are identified (step S13). In the example of FIG. 4, the classifier distinguishes between a lesion A, a lesion B, and normal (no lesion).

Training data for generating a trained model is also generated as 2D patches cut out from the tomographic images in a procedure similar to the procedure as described above. For example, a label indicating any one of the lesion A, lesion B, and normal is added to a 2D patch for training, and machine learning of the classifier is carried out with the labeled 2D patches for training.

Here, in an MR image using a linear MRI hepatographic imaging agent (gadoxetate sodium), a tumor in an organ (liver in a hepatobiliary phase in this case) is depicted darkly relative to the organ, and a blood vessel in an organ is also depicted darkly relative to the organ in a similar manner. Accordingly, in a case of identifying a tumor using the method of FIG. 4, a blood vessel region (e.g., normal region) may be erroneously identified as a tumor region.

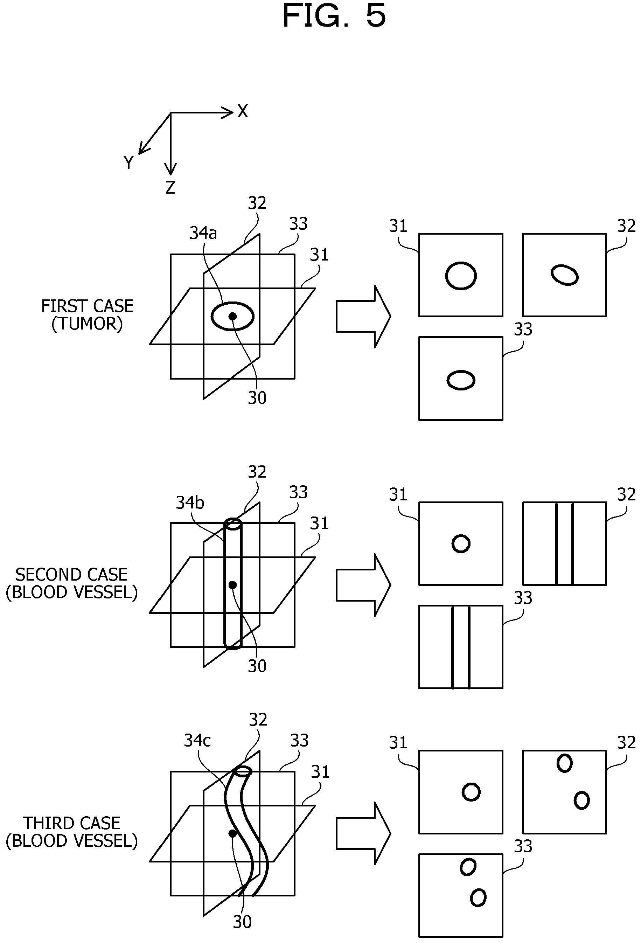

FIG. 5 is a second diagram illustrating a comparative example of the lesion identification process. Three-dimensional volume data may be generated from a tomographic image set including multiple tomographic images by interpolation processing. Additionally, by using such volume data, a tomographic image in a direction different from that of the original tomographic image may be generated. For example, a sagittal plane tomographic image or a coronal plane tomographic image may be generated based on an axial plane tomographic image set.

Then, it is conceivable to perform, on a certain identification target position, lesion identification using tomographic images in a plurality of directions instead of tomographic images in one direction as in the example of FIG. 4. In the example of FIG. 5, an axial plane tomographic image 31, a sagittal plane tomographic image 32, and a coronal plane tomographic image 33, each of which passes through a central position 30 of the identification target, are generated, and those three tomographic images (orthogonal three-plane images) are input to the classifier.

Since blood vessels may highly likely be depicted in different shapes in the tomographic images in the plurality of directions passing through the same central position 30, it is considered that the accuracy in identifying the tumor may be improved as compared to the method of FIG. 4. For example, in the first case illustrated in FIG. 5, a tumor 34a is present at the central position 30. Since tumors are often spherical, the tumor 34a in FIG. 5 is depicted in a circular shape or an oval shape in any of the axial plane tomographic image 31, the sagittal plane tomographic image 32, and the coronal plane tomographic image 33. On the other hand, since blood vessels are cylindrical, a blood vessel may be depicted in an elongated shape in at least one tomographic image in the plurality of directions. For example, in the second case illustrated in FIG. 5, a blood vessel 34b extends along the sagittal plane. In this case, while the blood vessel 34b is depicted in a circular shape or an oval shape in the axial plane tomographic image 31, the blood vessel 34b is depicted in an elongated shape in both of the sagittal plane tomographic image 32 and the coronal plane tomographic image 33. Therefore, it is highly likely that the tumor and the blood vessel are correctly identified between the first case and the second case.

However, cases where a blood vessel extends along a tomographic image as in the second case are rare. For example, like a blood vessel 34c in the third case illustrated in FIG. 5, a blood vessel often extends curvilinearly in reality. In the third case, the blood vessel 34c is depicted in a circular shape or an oval shape in any of the axial plane tomographic image 31, the sagittal plane tomographic image 32, and the coronal plane tomographic image 33. Therefore, in such a case, a blood vessel region (e.g., normal region) may be erroneously identified as a tumor region.

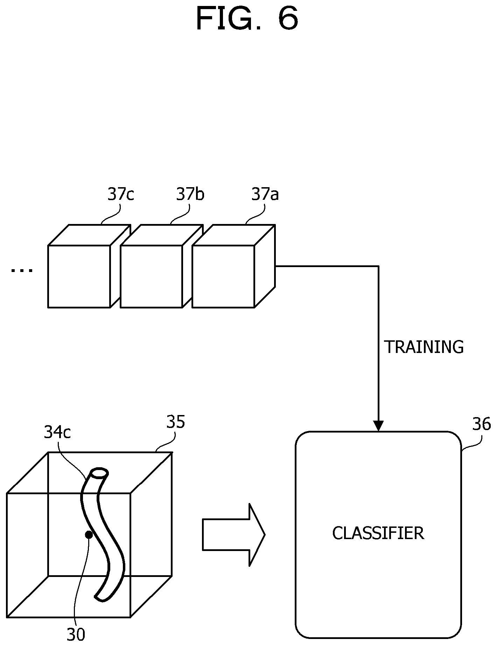

FIG. 6 is a third diagram illustrating a comparative example of the lesion identification process. In order to address such an erroneous identification problem as described above, a method of using three-dimensional data as input data to the classifier is conceivable. In FIG. 6, the lesion identification process is carried out in units of 3D patches of a certain size centered on the central position of the identification target. A 3D patch is a partial volume data obtained by cutting out a cubic area of a certain size from three-dimensional volume data.

FIG. 6 illustrates, as an example, a 3D patch 35 of a region including the blood vessel 34c illustrated in the third case of FIG. 5. This 3D patch 35 is generated by cutting out a cubic area having the same side as the 2D patch centered on the central position 30 of the identification target from the volume data generated based on the tomographic image set. By inputting such a 3D patch 35 to a classifier 36, it becomes possible to lower the possibility that a blood vessel region is erroneously identified as a tumor region even in a case where the blood vessel is not along any of the axial plane tomographic image 31, the sagittal plane tomographic image 32, and the coronal plane tomographic image 33.

However, training data for training the classifier 36 described above is also 3D patches 37a, 37b, 37c, and so on, which are three-dimensional volume data. Accordingly, as compared to the case of using 2D patches, a problem is raised that the number of parameters for training increases and training processing time increases as a result thereof. Furthermore, another problem is that a memory capacity needed for the training process increases. Moreover, a problem is raised that, at the time of the identification process using the classifier as well, identification processing time increases and a memory capacity needed for the identification process increases as compared to the case of using 2D patches.

Then, in the second embodiment, two-dimensional image data is used as the training data and the input data to the classifier, thereby improving the tumor identification accuracy while suppressing the time needed for the training process and the identification process.

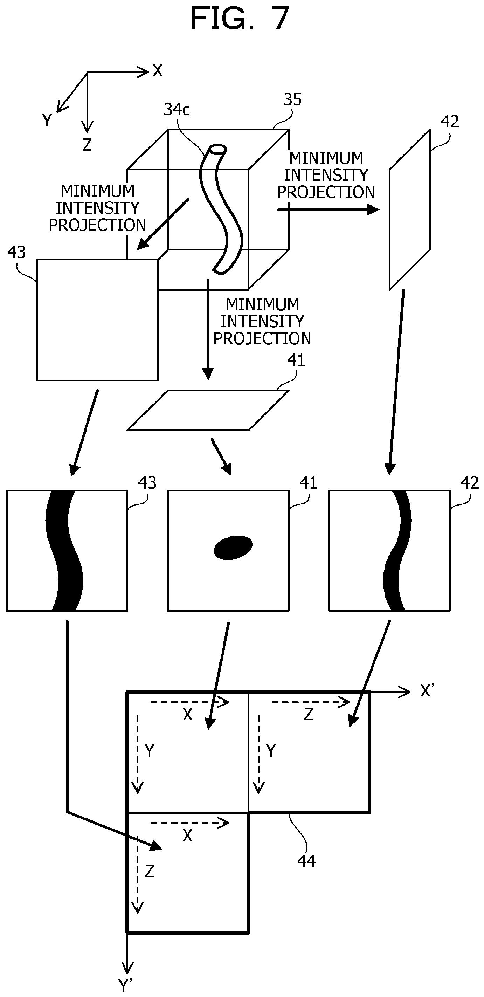

FIG. 7 is a diagram for explaining a process of generating identification input data according to the second embodiment. Note that, in the following descriptions, the right-to-left direction of the human body in a standing position is set as an X-axis, the back-to-front direction is set as a Y-axis, and the top-to-bottom direction is set as a Z-axis. In this case, the axial plane tomographic image is an image along an X-Y plane, the sagittal plane tomographic image is an image along a Y-Z plane, and the coronal plane tomographic image is an image along an X-Z plane.

In the present embodiment, minimum intensity projection images for the three axial directions based on 3D patches are used as input data for the classifier. As illustrated in FIG. 7, minimum intensity projection is carried out in the Z-axis direction for each pixel on the X-Y plane (axial plane) of the 3D patch 35, thereby generating a minimum intensity projection image 41 in the Z-axis direction is generated. Furthermore, the minimum intensity projection is carried out in the X-axis direction for each pixel on the Y-Z plane (sagittal plane) of the 3D patch 35, thereby generating a minimum intensity projection image 42 in the X-axis direction is generated. Moreover, the minimum intensity projection is carried out in the Y-axis direction for each pixel on the X-Z plane (coronal plane) of the 3D patch 35, thereby generating a minimum intensity projection image 43 in the Y-axis direction is generated.

Note that a pixel value of the minimum intensity projection image 41 in the Z-axis direction is obtained by the following calculation, for example. Here, n tomographic images (axial plane tomographic images in this case) are assumed to be generated from the 3D patch, and the pixel value of the coordinates (x, y) in the i-th tomographic image is assumed to be $g_i(x, y)$.

At this time, a pixel value h(x, y) at the coordinates (x, y) in a minimum intensity projection image h is calculated using the following equation (1). "min{...}" represents the minimum value among the values included in {...}. $h(x, y) = \min\{g_i(x, y)\}$, i = 1, 2, ..., n ... (1)

Note that a value of n is preferably determined such that a tumor of a desired size to be detected is included in the 3D patch cut out from the volume data. For example, in a case of enabling detection of a tumor larger than half the patch size (size of one side of the 3D patch) with the minimum size of the tumor to be detected set to r and a distance between tomographic images set to d, n is determined such that n*d/2 < r is satisfied.

For example, while the blood vessel 34c illustrated in FIG. 7 extends curvilinearly, the blood vessel is depicted in an elongated shape in at least one of the minimum intensity projection images 41 to 43 regardless of the shape of the blood vessel. In the example of FIG. 7, the blood vessel 34c is depicted in an oval shape in the minimum intensity projection image 41, whereas the blood vessel 34c is depicted in an elongated shape in the minimum intensity projection images 42 and 43. Therefore, by using such minimum intensity projection images in the three directions as input data, it becomes possible to lower the possibility that the blood vessel is erroneously identified as a tumor, and to improve the identification accuracy of the classifier.

Furthermore, in the present embodiment, the minimum intensity projection images in the three directions generated based on the 3D patch are combined as a combined 2D patch 44, which is one image. Note that, in FIG. 7, the X-axis and the Y-axis in the combined 2D patch 44 are indicated as an X'-axis and a Y'-axis, respectively, to avoid confusion.

As illustrated in FIG. 7, in the combined 2D patch 44, the minimum intensity projection image 41 in the Z-axis direction and the minimum intensity projection image 42 in the X-axis direction are adjacently combined with their Y coordinates coincide with each other. Furthermore, the minimum intensity projection image 41 in the Z-axis direction and the minimum intensity projection image 43 in the Y-axis direction are adjacently combined with their X coordinates coincide with each other.

In this case, the X and Y coordinates in the minimum intensity projection image 41 are directly used as the X' and Y' coordinates in the combined 2D patch 44, respectively. Furthermore, the Y coordinate in the minimum intensity projection image 42 is directly used as the Y' coordinate in the combined 2D patch 44, and a value obtained by adding the patch size to the Z coordinate in the minimum intensity projection image 42 is used as the X' coordinate in the combined 2D patch 44. Moreover, the X coordinate in the minimum intensity projection image 43 is directly used as the X' coordinate in the combined 2D patch 44, and a value obtained by adding the patch size to the Z coordinate in the minimum intensity projection image 43 is used as the Y' coordinate in the combined 2D patch 44.

In this manner, the combined 2D patch, which is two-dimensional image data, is input to the classifier. Furthermore, training data to be used for training of the classifier is also the combined 2D patch obtained by combining the minimum intensity projection images in the three directions generated from the original 3D patch. For example, a label indicating a tumor or normal is added to combined 2D patches for training, and training of the classifier is carried out using those combined 2D patches. As a result, as compared to the case of using the 3D patch as the training data, it becomes possible to shorten the time needed for the training process, and to reduce the memory capacity needed for the training process. Furthermore, for the identification process using the classifier obtained by the training as well, it becomes possible to shorten the processing time and to reduce the needed memory capacity.

Note that the minimum intensity projection image generated based on the 3D patch is not necessarily an image projected in the direction along the coordinate axis. However, individual minimum intensity projection images are preferably images projected in mutually orthogonal directions. Furthermore, two minimum intensity projection images projected in two mutually orthogonal directions may be generated based on the 3D patch. Even in this case as well, the blood vessel in the 3D patch is depicted in an elongated shape extending from one side of the image to the same side or another side in at least one of the minimum intensity projection images.

Furthermore, inside the liver, both of the tumor and the blood vessel are depicted darker than their surroundings. Accordingly, the combined 2D patch is generated by combining the minimum intensity projection images. However, depending on the lesion to be identified, it may be depicted brighter than surrounding areas. In this case, the combined patch may be generated by combining maximum intensity projection images.

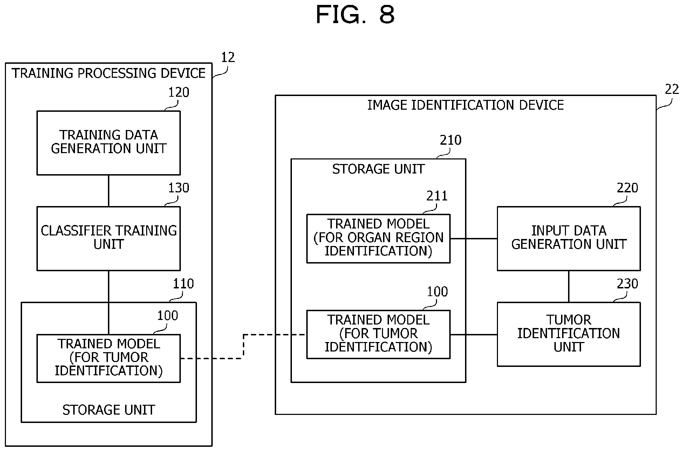

Next, FIG. 8 is a diagram illustrating an exemplary configuration of processing functions of the training processing device and the image identification device.

The training processing device 12 includes a storage unit 110, a training data generation unit 120, and a classifier training unit 130.

The storage unit 110 is a storage area secured in a storage device included in the training processing device 12. The storage unit 110 stores data indicating the trained model 100 for tumor identification (weight of the trained model 100).

Processing of the training data generation unit 120 and the classifier training unit 130 is implemented by execution of a predetermined program by a processor included in the training processing device 12, for example. The training data generation unit 120 generates training data for training the classifier for tumor identification based on one or more tomographic image sets obtained by imaging using the MRI apparatus 11. The aforementioned combined 2D patch is generated as this training data. The classifier training unit 130 carries out machine learning using the generated combined 2D patch for training, thereby generating the trained model 100 indicating the classifier for tumor identification. The weight of the generated trained model 100 is stored in the storage unit 110.

The image identification device 22 includes a storage unit 210, an input data generation unit 220, and a tumor identification unit 230.

The storage unit 210 is a storage area secured in a storage device included in the image identification device 22, such as the RAM 202, the HDD 203, or the like. The storage unit 210 stores a weight of a trained model 211 for organ region identification and the weight of the trained model 100 for tumor identification.

The trained model 211 is a model indicating a classifier for identifying a region of an organ (liver in the present embodiment) in a tomographic image set, and is generated in advance by machine learning. The weight of the trained model 100 is generated by the training processing device 12, read into the image identification device 22 via a network or via a portable recording medium, for example, and stored in the storage unit 210.

Processing of the input data generation unit 220 and the tumor identification unit 230 is implemented by the processor 201 executing a predetermined program, for example. The input data generation unit 220 generates a combined 2D patch for each 3D patch position based on the tomographic image set obtained by imaging using the MRI apparatus 21. In this generation process, a classifier for organ region identification based on the trained model 211 is used. The tumor identification unit 230 inputs the combined 2D patch generated by the input data generation unit 220 to the classifier based on the trained model 100, and identifies whether the 3D patch corresponding to the combined 2D patch is a tumor or normal.

Next, a training data generation process performed by the training processing device 12 will be described.

With regard to an MR image of an organ (liver), inside of the organ may become darker as it approaches a boundary with an extra-organ region from the center, and gradation may occur in a normal region other than a tumor near the boundary. Accordingly, it may not be possible to correctly distinguish between normal or a tumor near the boundary. Then, the training data generation unit 120 includes not only data from the center side region at some distance from the boundary but also a sufficient number of pieces of data from the region near the boundary in the training data of each of a tumor label and a normal label. This makes it possible to generate a classifier capable of correctly distinguishing between a tumor and normal even in a region near the boundary.

Furthermore, the training data generation unit 120 ensures that a sufficient number of pieces of data from a region including a blood vessel is included in the training data for the normal label. This makes it possible to generate a classifier capable of accurately distinguishing between a blood vessel and a tumor. Moreover, with regard to the training data for the tumor label, the training data generation unit 120 generates training data from 3D patches selected such that a proportion of the tumor region varies. As a result, a classifier capable of correctly identifying tumors of various sizes may be achieved.

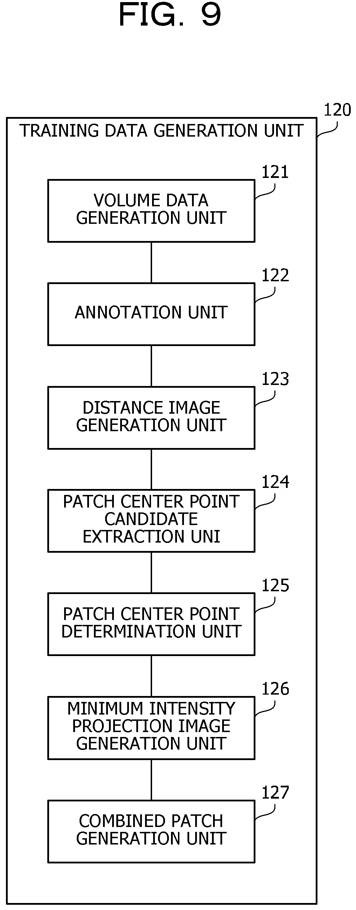

FIG. 9 is a diagram illustrating an exemplary internal configuration of the training data generation unit. As illustrated in FIG. 9, the training data generation unit 120 includes a volume data generation unit 121, an annotation unit 122, a distance image generation unit 123, a patch center point candidate extraction unit 124, a patch center point determination unit 125, a minimum intensity projection image generation unit 126, and a combined patch generation unit 127.

The volume data generation unit 121 generates three-dimensional volume data based on the tomographic image set obtained by the imaging using the MRI apparatus 11.

The annotation unit 122 annotates each voxel of the volume data into an intra-organ region and an extra-organ region as well as into a tumor region and a normal region.

The distance image generation unit 123 generates a three-dimensional distance image based on the annotation result. As this distance image, a "first distance image" indicating a distance from the intra-organ region side to the organ boundary (boundary with extra-organ region) and a "second distance image" indicating a distance from the normal region side to the tumor are generated.

The patch center point candidate extraction unit 124 extracts, from the volume data, center point candidates of a normal 3D patch and center point candidates of a tumor 3D patch based on the first distance image and the second distance image. The normal 3D patch is a 3D patch corresponding to a combined 2D patch labeled "normal". Furthermore, the tumor 3D patch is a 3D patch corresponding to a combined 2D patch labeled "tumor". Through the processing of the patch center point candidate extraction unit 124, candidates for each of the normal 3D patch and the tumor 3D patch are evenly extracted from both of the organ region near the boundary and the center side region at some distance from the boundary within the intra-organ region.

The patch center point determination unit 125 determines individual final center points of the normal 3D patch and tumor 3D patch based on statistical information regarding luminance in the corresponding 3D patch from among the extracted center point candidates. Through the processing of the patch center point determination unit 125, normal 3D patches are selected such that a sufficient number of 3D patches including blood vessels is included, and the tumor 3D patch is selected such that the proportion of the tumor region varies.

The minimum intensity projection image generation unit 126 generates minimum intensity projection images in three axial directions for each of the normal 3D patch and the tumor 3D patch.

The combined patch generation unit 127 combines the minimum intensity projection images in the three axial directions to generate a combined 2D patch for each of the normal 3D patch and the tumor 3D patch.

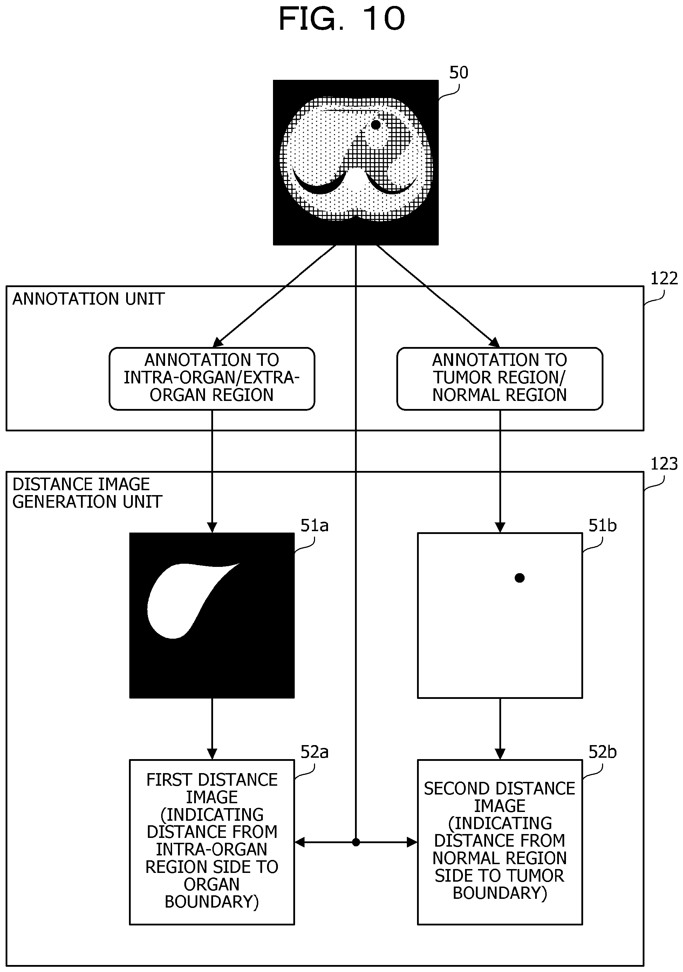

FIG. 10 is a diagram for explaining a process of annotation and distance image generation.

The annotation unit 122 obtains three-dimensional volume data 50 generated based on the tomographic image set from the volume data generation unit 121. The annotation unit 122 annotates each voxel of the generated volume data 50 into an intra-organ region and an extra-organ region. This annotation may be executed using, for example, a classifier that identifies an organ region in the tomographic image set. This classifier is based on a trained model (model equivalent to the trained model 100 in FIG. 8) generated in advance by machine learning.

Furthermore, the annotation unit 122 annotates each voxel of the generated volume data 50 into a tumor region and a normal region. This annotation is manually carried out by a user, for example.

The distance image generation unit 123 generates a binary image 51a that distinguishes between the intra-organ region and the extra-organ region and a binary image 51b that distinguishes between the tumor region and the normal region based on the annotation result. The binary image 51a is a three-dimensional binary image in which a voxel in the intra-organ region is set to "1" and a voxel in the extra-organ region is set to "0" among the voxels of the volume data 50. The binary image 51b is a three-dimensional binary image in which a voxel in the normal region is set to "1" and a voxel in the tumor region is set to "0" among the voxels of the volume data 50.

Next, the distance image generation unit 123 generates a first distance image 52a based on the binary image 51a and the volume data 50. This first distance image 52a is a three-dimensional distance image in which a distance from the intra-organ region side to the organ boundary is set for each voxel of the volume data 50. For example, in the first distance image 52a, a distance "0" is set for each voxel in the extra-organ region, and a value corresponding to a distance to the organ boundary is set for each voxel in the intra-organ region. In the intra-organ region, a distance value increases toward the center part.

Furthermore, the distance image generation unit 123 generates a second distance image 52b based on the binary image 51b and the volume data 50. This second distance image 52b is a three-dimensional distance image in which a distance from the normal region side to the boundary with the tumor is set for each voxel of the volume data 50. For example, in the second distance image 52b, a distance "0" is set for each voxel in the tumor region, and a value corresponding to a distance to the boundary with the tumor is set for each voxel in the normal region. In the normal region, a distance value increases as it separates farther from the tumor region.

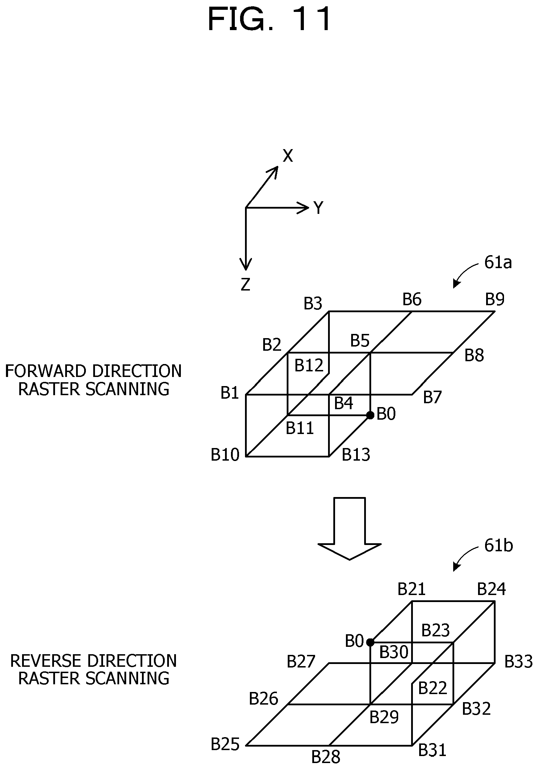

FIG. 11 is a diagram illustrating a first example of a distance image generation method. According to the first example illustrated in FIG. 11, a three-dimensional mask is moved from one end of the volume data 50 to the opposite end facing thereto, thereby calculating a distance of each voxel of the volume data 50. In FIG. 11, as an example, it is assumed that the mask is subject to raster scanning on a tomographic image on the X-Y plane (axial plane tomographic image) and then sequentially moved in the Z-axis direction.

First, a mask 61a for forward scanning is used. The mask 61a includes a voxel B0 to be subject to distance calculation, and 13 voxels B1 to B13 existing therearound. Furthermore, as an initial setting for a distance image, the distance image generation unit 123 sets "0" to a voxel in which a value of the corresponding binary image is "0", and sets M to a voxel in which a value of the corresponding binary image is "1" among the voxels of the volume data 50. A value larger than the expected maximum distance value is set as M.

The distance image generation unit 123 carries out the following distance calculation while moving the mask 61a in such a manner that the position of the voxel B0 is subject to raster scanning in the X direction starting from the origin point of the tomographic image on the X-Y plane and then such raster scanning moves in the Z-axis direction. When the distance value of the voxel B0 is not "0", the distance image generation unit 123 extracts the minimum value from the distance values of the voxels B1 to B13 therearound, and updates the distance value of the voxel B0 with a value obtained by adding "1" to the minimum value. On the other hand, when the distance value of the voxel B0 is "0", the distance image generation unit 123 keeps the distance value without updating it.

When the processing described above has been performed up to the final position, next, a mask 61b for operation in the reverse direction is used. The mask 61b includes a voxel B0 to be subject to distance calculation, and 13 voxels B21 to B33 existing therearound. The distance image generation unit 123 carries out the following distance calculation while moving the mask 61b in such a manner that the position of the voxel B0 moves in the direction opposite to the processing described above from the final position. When the distance value of the voxel B0 is not "0", the distance image generation unit 123 extracts the minimum value from the distance values of the voxels B21 to B33 therearound, and updates the distance value of the voxel B0 with a value obtained by adding "1" to the minimum value. On the other hand, when the distance value of the voxel B0 is "0", the distance image generation unit 123 keeps the distance value without updating it.

Note that, in the process of generating the first distance image according to the method described above, a distance value of a voxel not included in the volume data 50 among the voxels B1 to B13 and B21 to B33 is set to "0". Meanwhile, in the process of generating the second distance image according to the method described above, a distance value of a voxel not included in the volume data 50 among the voxels B1 to B13 and B21 to B33 is set to M.

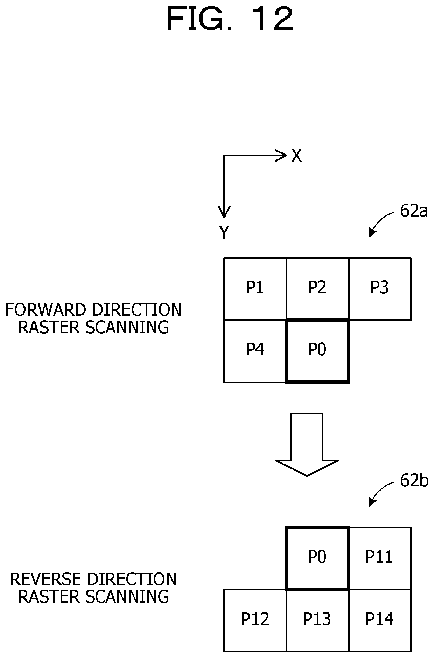

FIG. 12 is a diagram illustrating a second example of the distance image generation method. According to the second example illustrated in FIG. 12, a two-dimensional mask is used to calculate a distance, thereby simplifying the distance calculation process. According to the method illustrated in FIG. 12, although the distance calculation accuracy lowers as compared to the method illustrated in FIG. 11, the time needed for the distance calculation process may be shortened.

Here, in a similar manner to FIG. 11, it is assumed that the mask is subject to raster scanning on a tomographic image on the X-Y plane (axial plane tomographic image) and then sequentially moved in the Z-axis direction. First, a mask 62a for forward scanning is used. The mask 62a includes a pixel P0 to be subject to distance calculation, and four pixels P1 to P4 adjacent thereto. Furthermore, as an initial setting for a distance image, the distance image generation unit 123 sets "0" to a pixel in which a value of the corresponding binary image is "0", and sets M to a pixel in which a value of the corresponding binary image is "1" among the pixels of each tomographic image (e.g., voxels) of the volume data 50. A value larger than the expected maximum distance value is set as M.

The distance image generation unit 123 carries out the following distance calculation while moving the mask 62a in such a manner that the position of the pixel P0 is subject to raster scanning in the X direction starting from the origin point of the tomographic image on the X-Y plane and then such raster scanning moves in the Z-axis direction. When the distance value of the pixel P0 is not "0", the distance image generation unit 123 extracts the minimum value from the distance values of the pixels P1 to P4 therearound, and updates the distance value of the pixel P0 with a value obtained by adding "1" to the minimum value. On the other hand, when the distance value of the pixel P0 is "0", the distance image generation unit 123 keeps the distance value without updating it.

When the processing described above has been performed up to the final position, next, a mask 62b for operation in the reverse direction is used. The mask 62b includes a pixel P0 to be subject to distance calculation, and four pixels P11 to P14 adjacent thereto. The distance image generation unit 123 carries out the following distance calculation while moving the mask 62b in such a manner that the position of the pixel P0 moves in the direction opposite to the processing described above from the final position. When the distance value of the pixel P0 is not "0", the distance image generation unit 123 extracts the minimum value from the distance values of the pixels P11 to P14 therearound, and updates the distance value of the pixel P0 with a value obtained by adding "1" to the minimum value. On the other hand, when the distance value of the pixel P0 is "0", the distance image generation unit 123 keeps the distance value without updating it.

Note that, in the process of generating the first distance image according to the method described above, a distance value of a pixel not included in the volume data 50 among the pixels P1 to P4 and P11 to P14 is set to "0". Meanwhile, in the process of generating the second distance image according to the method described above, a distance value of a pixel not included in the volume data 50 among the pixels P1 to P4 and P11 to P14 is set to M.

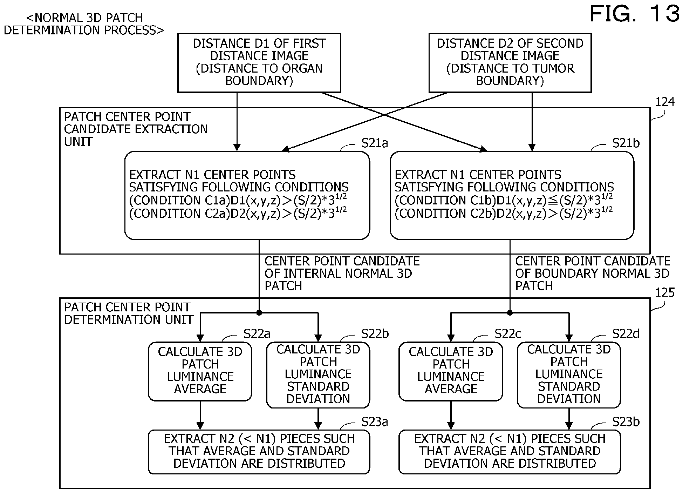

FIG. 13 is a diagram illustrating a processing procedure of normal 3D patch determination. Through the processing of the patch center point candidate extraction unit 124 and the patch center point determination unit 125, a normal 3D patch corresponding to training data (combined 2D patch) labeled "normal" is determined. In this determination process, an "internal normal 3D patch" existing in the center side region at some distance from the boundary with the outside of the organ and a "boundary normal 3D patch" existing in the region near boundary are separately determined within the intra-organ region.

In the following descriptions, a distance for the voxel with the coordinates $(x, y, z)$ in the first distance image is assumed to be $D1(x, y, z)$. Furthermore, a distance for the voxel with the coordinates $(x, y, z)$ in the second distance image is assumed to be $D2(x, y, z)$.

First, the patch center point candidate extraction unit 124 extracts N1 voxels satisfying both of the following conditions C1a and C2a from the voxels of the organ region in the volume data as center point candidates of the internal normal 3D patch (step S21a). Note that S represents a patch size (length of one side of the 3D patch). As a specific process, the patch center point candidate extraction unit 124 extracts all the voxels satisfying both of the conditions C1a and C2a from the organ region, and then randomly extracts N1 voxels from among them.

$$\text{(Condition C1a) } D1(x, y, z) > (S/2)*3^{1/2}$$

$$\text{(Condition C2a) } D2(x, y, z) > (S/2)*3^{1/2}$$

The condition C1a indicates that the distance value of the first distance image is larger than half the diagonal length of the 3D patch. For example, the center point (voxel) satisfying the condition C1a exists in the center side region at some distance from the boundary with the outside of the organ. Furthermore, the center point satisfying the condition C2a indicates that the distance value of the second distance image is larger than half the diagonal length of the 3D patch. For example, the center point (voxel) satisfying the condition C2a exists in a region (normal region) outside the tumor region. Accordingly, through the process described above, N1 center point candidates of the internal tumor 3D patch are extracted.

Furthermore, the patch center point candidate extraction unit 124 extracts N1 voxels satisfying both of the following conditions C1b and C2b from the voxels of the organ region in the volume data as center point candidates of the boundary normal 3D patch (step S21b). As a specific process, the patch center point candidate extraction unit 124 extracts all the voxels satisfying both of the conditions C1b and C2b from the organ region, and then randomly extracts N1 voxels from among them.

$$\text{(Condition C1b)} \quad D1(x, y, z) \leq (S/2)*3^{1/2}$$

$$\text{(Condition C2b)} \quad D2(x, y, z) > (S/2)*3^{1/2}$$

The condition C1b indicates that the distance value of the first distance image is equal to or smaller than the diagonal length of the 3D patch. For example, the center point (voxel) satisfying the condition C1b exists in the region near the boundary with the outside of the organ. Furthermore, the condition C2b is the same as the condition C2a, and the center point (voxel) satisfying the condition C2b exists in a region outside the tumor region (normal region). Accordingly, through the process described above, N1 center point candidates of the boundary tumor 3D patch are extracted.

Through the process of steps S21a and S21b described above, center point candidates of the normal 3D patch are evenly extracted from both of the region at some distance from the boundary and the region near the boundary. Next, the patch center point determination unit 125 narrows down the center points of the internal normal 3D patches to N2 (< N1) pieces, and also narrows down the center points of the boundary normal 3D patches to N2 pieces. Through this narrowing-down process, the final normal 3D patches are determined based on statistical information regarding luminance in the 3D patches in such a manner that the luminance distribution varies among the 3D patches. As a result, a sufficient number of normal 3D patches in regions including blood vessels is included in the determined normal 3D patches.

Note that, although the numbers of ultimately determined internal normal 3D patches and boundary normal 3D patches are assumed to be the same here, they are not necessarily the same number, and may be approximately the same number. Furthermore, those ratios may be intentionally changed for sensitivity adjustment or the like.

For example, the patch center point determination unit 125 carries out the narrowing-down process according to the following procedure.

The patch center point determination unit 125 calculates, for each of the center point candidates extracted in step S21a, an average luminance value of each voxel within the corresponding 3D patch (step S22a) and a luminance standard deviation (step S22b). Based on the calculation results in steps S22a and S22b, the patch center point determination unit 125 extracts N2 center points corresponding to the final internal normal 3D patches from the center point candidates in such a manner that the average luminance values and the luminance standard deviations are distributed (step S23a).

As described above, in an MR image, a blood vessel region is depicted darker than surrounding organ regions. Accordingly, with internal normal 3D patches having mutually different luminance distributions extracted through the procedure described above, regions including blood vessels and regions including no blood vessel are to be extracted in a well-balanced manner. As a result, it becomes possible to generate a classifier capable of accurately determining a blood vessel region as normal.

Furthermore, the patch center point determination unit 125 calculates, for each of the center point candidates extracted in step S21b, an average luminance value of each voxel within the corresponding 3D patch (step S22c) and a luminance standard deviation (step S22d). Based on the calculation results in steps S22c and S22d, the patch center point determination unit 125 extracts N2 center points corresponding to the final boundary normal 3D patches from the center point candidates in such a manner that the average luminance values and the luminance standard deviations are distributed (step S23b).

Accordingly, boundary normal 3D patches having mutually different luminance distributions are extracted, whereby regions including blood vessels and regions including no blood vessel are to be extracted in a well-balanced manner. As a result, it becomes possible to generate a classifier capable of accurately determining a blood vessel region as normal.

Note that only one of the average luminance value and the luminance standard deviation may be used in the process of narrowing down N2 center point candidates from N1 center point candidates. However, for example, the average values may largely differ in some cases even when the standard deviation is the same, and thus regions including blood vessels and regions including no blood vessel may be uniformly extracted by using both of them.

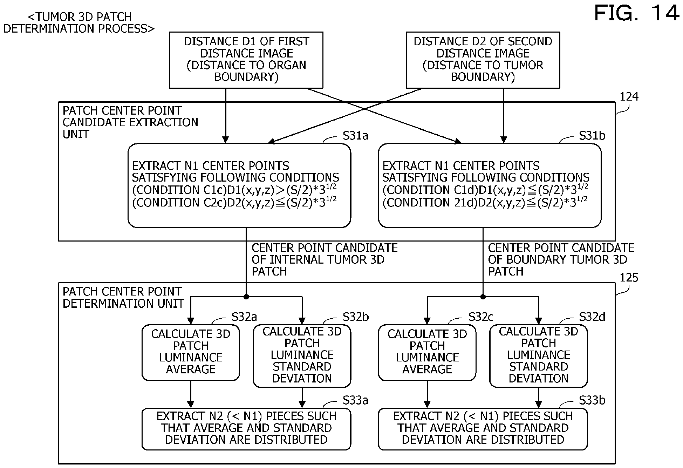

FIG. 14 is a diagram illustrating a processing procedure of tumor 3D patch determination. Through the processing of the patch center point candidate extraction unit 124 and the patch center point determination unit 125, a tumor 3D patch corresponding to training data (combined 2D patch) labeled "tumor" is determined. The tumor 3D patch is determined through a procedure similar to that for the normal 3D patch. For example, in this determination process, an "internal tumor 3D patch" existing in the center side region at some distance from the boundary with the outside of the organ and a "boundary tumor 3D patch" existing in the region near boundary are separately determined within the intra-organ region.

First, the patch center point candidate extraction unit 124 extracts N1 voxels satisfying both of the following conditions C1c and C2c from the voxels of the organ region in the volume data as center point candidates of the internal tumor 3D patch (step S31a). As a specific process, the patch center point candidate extraction unit 124 extracts all the voxels satisfying both of the conditions C1c and C2c from the organ region, and then randomly extracts N1 voxels from among them.

$$\text{(Condition C1c) } D1(x, y, z) > (S/2)*3^{1/2}$$

$$\text{(Condition C2c) } D2(x, y, z) \leq (S/2)*3^{1/2}$$

The condition C1c indicates that the distance value of the first distance image is larger than half the diagonal length of the 3D patch. For example, the center point (voxel) satisfying the condition C1c exists in the center side region at some distance from the boundary with the outside of the organ. Furthermore, the center point satisfying the condition C2c indicates that the distance value of the second distance image is equal to or smaller than half the diagonal length of the 3D patch. For example, the center point (voxel) satisfying the condition C2c exists in the tumor region.

Furthermore, the patch center point candidate extraction unit 124 extracts N1 voxels satisfying both of the following conditions C1d and C2d from the voxels of the organ region in the volume data as center point candidates of the boundary tumor 3D patch (step S31b). As a specific process, the patch center point candidate extraction unit 124 extracts all the voxels satisfying both of the conditions C1d and C2d from the organ region, and then randomly extracts N1 voxels from among them.

$$\text{(Condition C1d) } D1(x, y, z) \leq (S/2)*3^{1/2}$$

$$\text{(Condition C2d) } D2(x, y, z) \leq (S/2)*3^{1/2}$$

The condition C1d indicates that the distance value of the first distance image is equal to or smaller than the diagonal length of the 3D patch. For example, the center point (voxel) satisfying the condition C1d exists in the region near the boundary with the outside of the organ. Furthermore, the condition C2d is the same as the condition C2c, and the center point (voxel) satisfying the condition C2d exists the tumor region.

Through the process of steps S31a and S31b described above, center point candidates of the tumor 3D patch are evenly extracted from both of the region at some distance from the boundary and the region near the boundary. Next, the patch center point determination unit 125 narrows down the center points of the internal tumor 3D patches to N2 (< N1) pieces, and also narrows down the center points of the boundary tumor 3D patches to N2 pieces. Through this narrowing-down process, the final tumor 3D patches are determined based on statistical information regarding luminance in the 3D patches in such a manner that the luminance distribution varies among the 3D patches. As a result, the final tumor 3D patches are determined in such a manner that the size of the tumor region varies among the determined 3D patches.

Note that, although the numbers of ultimately determined internal tumor 3D patches and boundary tumor 3D patches are assumed to be the same here, they are not necessarily the same number, and may be approximately the same number. Furthermore, those ratios may be intentionally changed for sensitivity adjustment or the like.

For example, the patch center point determination unit 125 carries out the narrowing-down process according to the following procedure.

The patch center point determination unit 125 calculates, for each of the center point candidates extracted in step S31a, an average luminance value of each voxel within the corresponding 3D patch (step S32a) and a luminance standard deviation (step S32b). Based on the calculation results in steps S32a and S32b, the patch center point determination unit 125 extracts N2 center points corresponding to the final internal tumor 3D patches from the center point candidates in such a manner that the average luminance values and the luminance standard deviations are distributed (step S33a).

As described above, in an MR image, a tumor region is depicted darker than surrounding organ regions. Accordingly, with internal tumor 3D patches having mutually different luminance distributions extracted through the procedure described above, internal tumor 3D patches including various forms of tumors, such as tumors of different sizes, tumors with different internal gradation conditions, and the like, are to be extracted. As a result, it becomes possible to generate a classifier capable of accurately determining a tumor region as a tumor.

Furthermore, the patch center point determination unit 125 calculates, for each of the center point candidates extracted in step S31b, an average luminance value of each voxel within the corresponding 3D patch (step S32c) and a luminance standard deviation (step S32d). Based on the calculation results in steps S32c and S32d, the patch center point determination unit 125 extracts N2 center points corresponding to the final boundary tumor 3D patches from the center point candidates in such a manner that the average luminance values and the luminance standard deviations are distributed (step S33b).

Accordingly, boundary tumor 3D patches having mutually different luminance distributions are extracted, whereby internal tumor 3D patches including various forms of tumors are to be extracted. As a result, it becomes possible to generate a classifier capable of accurately determining a tumor region as a tumor.

Note that, in a similar manner to the normal 3D patch determination process, in the tumor 3D patch determination process as well, only one of the average luminance value and the luminance standard deviation may be used in the process of narrowing down N2 center point candidates from N1 center point candidates. However, for example, the average values may largely differ in some cases even when the standard deviation is the same, and thus regions including various forms of tumors may be extracted by using both of them.

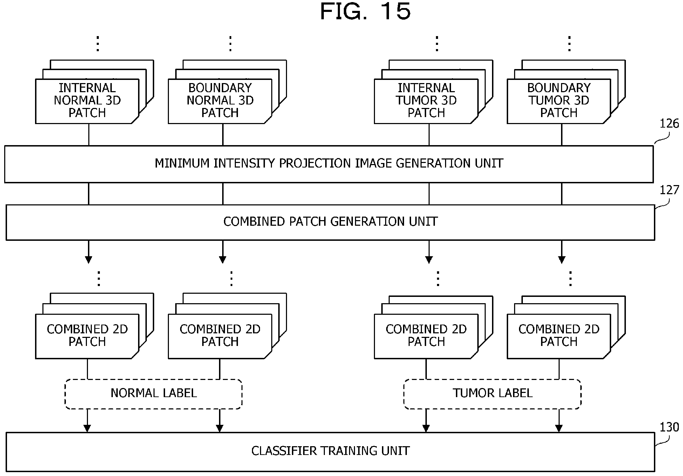

FIG. 15 is a diagram for explaining training data input to the classifier training unit.

For each of the internal normal 3D patches extracted by the patch center point determination unit 125, the minimum intensity projection image generation unit 126 generates minimum intensity projection images in the three axial directions, and the combined patch generation unit 127 combines the generated minimum intensity projection images to generate a combined 2D patch. Furthermore, for each of the boundary normal 3D patches extracted by the patch center point determination unit 125 as well, the minimum intensity projection image generation unit 126 generates minimum intensity projection images in the three axial directions, and the combined patch generation unit 127 combines the generated minimum intensity projection images to generate a combined 2D patch. A normal label is added to those combined 2D patches.

Moreover, for each of the internal tumor 3D patches extracted by the patch center point determination unit 125, the minimum intensity projection image generation unit 126 generates minimum intensity projection images in the three axial directions, and the combined patch generation unit 127 combines the generated minimum intensity projection images to generate a combined 2D patch. Furthermore, for each of the boundary tumor 3D patches extracted by the patch center point determination unit 125 as well, the minimum intensity projection image generation unit 126 generates minimum intensity projection images in the three axial directions, and the combined patch generation unit 127 combines the generated minimum intensity projection images to generate a combined 2D patch. A tumor label is added to those combined 2D patches.

In this manner, the combined 2D patches to which the normal labels are added and the combined 2D patches to which the tumor labels are added are generated and input to the classifier training unit 130. The classifier training unit 130 uses those combined 2D patches as training data to generate, by machine learning, a trained model of a classifier that distinguishes between normal and a tumor for each of the combined 2D patches (e.g., each 3D patch).

Practically, a plurality of tomographic image sets is used to generate, through the process described above, the combined 2D patches to which the normal labels are added and the combined 2D patches to which the tumor labels are added, which are input to the classifier training unit 130.

Since the minimum intensity projection images in the three axial directions are used as the training data, it becomes possible to generate a highly accurate classifier that is less likely to erroneously identify a blood vessel as a tumor. Furthermore, since the training data input to the classifier training unit 130 is 2D data instead of 3D data, it becomes possible to shorten the time needed for the training process, and to reduce the memory capacity needed for the training process as compared to the case of using the 3D data.

Note that the combined 2D patches to which the normal labels are added and the combined 2D patches to which the tumor labels are added are generated in the same number or approximately the same number, and are input to the classifier training unit 130. However, the ratio between the number of inputs of the combined 2D patches to which the normal labels are added and the number of inputs of the combined 2D patches to which the tumor labels are added may be intentionally changed for sensitivity adjustment or the like.

Furthermore, the machine learning using the combined 2D patches based on the internal normal 3D patches and the internal tumor 3D patches and the machine learning using the combined 2D patches based on the boundary normal 3D patches and the boundary tumor 3D patches may be separately carried out. In this case, a separate classifier is generated by each machine learning. Then, at the time of the identification process, it is determined whether the 3D patch that is the basis of the combined 2D patch exists in the center side region at some distance from the organ boundary or exists near the organ boundary, and the identification process is performed using the classifier corresponding to the determination result.

Next, processing of the training processing device 12 will be described using a flowchart.

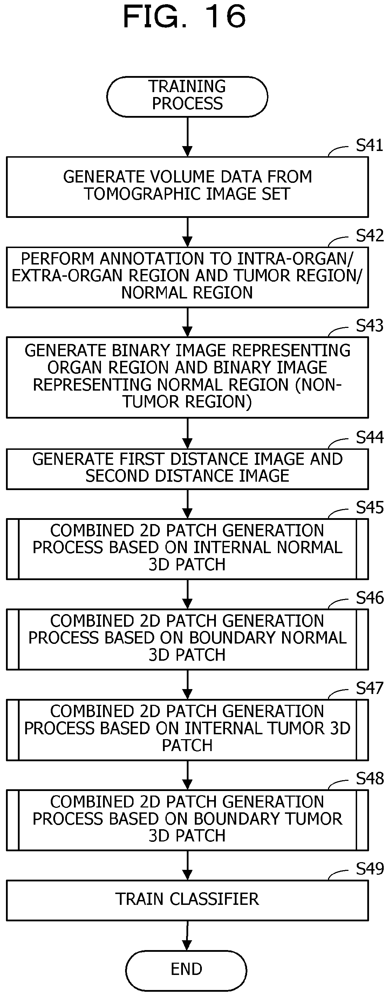

FIG. 16 is an exemplary flowchart illustrating a processing procedure of the entire training process.

[Step S41] The volume data generation unit 121 obtains a tomographic image set from the MRI apparatus 11. The volume data generation unit 121 generates three-dimensional volume data based on the obtained tomographic image set.

[Step S42] The annotation unit 122 annotates each voxel of the generated volume data into an intra-organ region and an extra-organ region as well as into a tumor region and a normal region.

[Step S43] The distance image generation unit 123 generates a first binary image representing the intra-organ region based on the annotation result of the intra-organ region and the extra-organ region. This first binary image is a three-dimensional binary image in which a voxel in the intra-organ region is set to "1" and a voxel in the extra-organ region is set to "0".

Furthermore, the distance image generation unit 123 generates a second binary image representing the normal region (non-tumor region) based on the annotation result of the tumor region and the normal region. This second binary image is a three-dimensional binary image in which a voxel in the normal region is set to "1" and a voxel in the tumor region is set to "0".

[Step S44] The distance image generation unit 123 generates a first distance image based on the first binary image and the volume data. This first distance image is a three-dimensional distance image in which a distance "0" is set for each voxel in the extra-organ region and a value corresponding to a distance to the organ boundary is set for each voxel in the intra-organ region.

Furthermore, the distance image generation unit 123 generates a second distance image based on the second binary image and the volume data. This second distance image is a three-dimensional distance image in which a distance "0" is set for each voxel in the tumor region and a value corresponding to a distance to the boundary with the tumor is set for each voxel in the normal region.

[Step S45] A process of generating a combined 2D patch based on an internal normal 3D patch is performed using the first distance image and the second distance image.

[Step S46] A process of generating a combined 2D patch based on a boundary normal 3D patch is performed using the first distance image and the second distance image.

[Step S47] A process of generating a combined 2D patch based on an internal tumor 3D patch is performed using the first distance image and the second distance image.

[Step S48] A process of generating a combined 2D patch based on a boundary tumor 3D patch is performed using the first distance image and the second distance image.

Note that the processing order of steps S45 to S48 is not limited to the order described above. Furthermore, the individual processing of steps S45 to S48 may be executed in parallel.

[Step S49] The classifier training unit 130 carries out machine learning using the combined 2D patches as training data, and generates the trained model 100 of the classifier that distinguishes between normal and a tumor. For example, the classifier training unit 130 generates the trained model 100 by machine learning using a neural network. The classifier training unit 130 stores the weight of the generated trained model 100 in the storage unit 110.

Note that, practically, a plurality of tomographic image sets is used to execute the process of steps S41 to S48, and the machine learning using the obtained combined 2D patches is carried out in step S49.

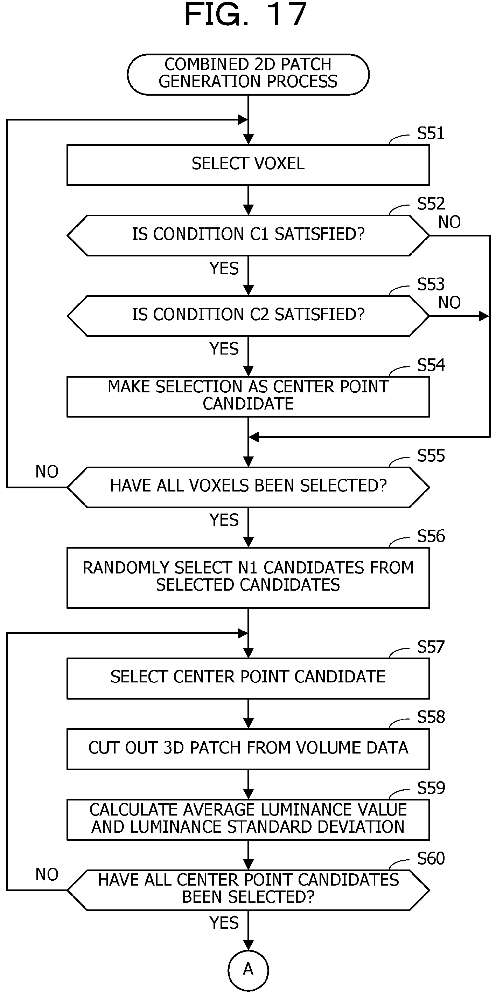

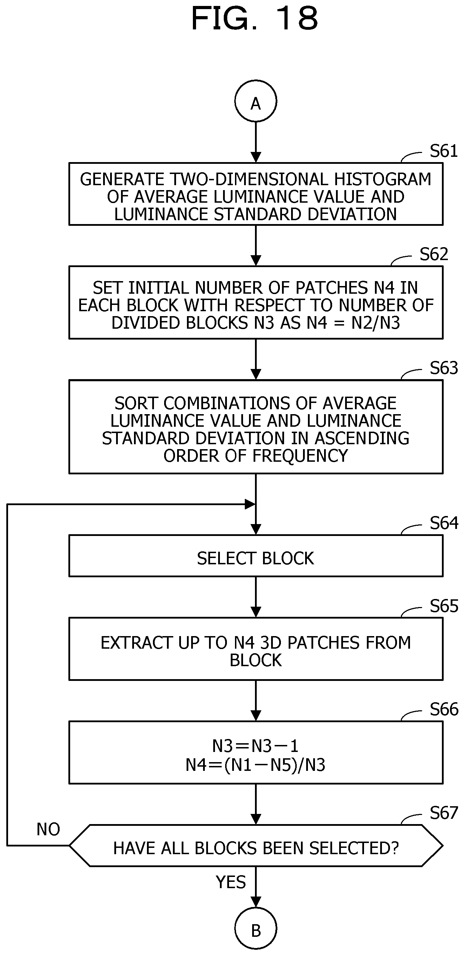

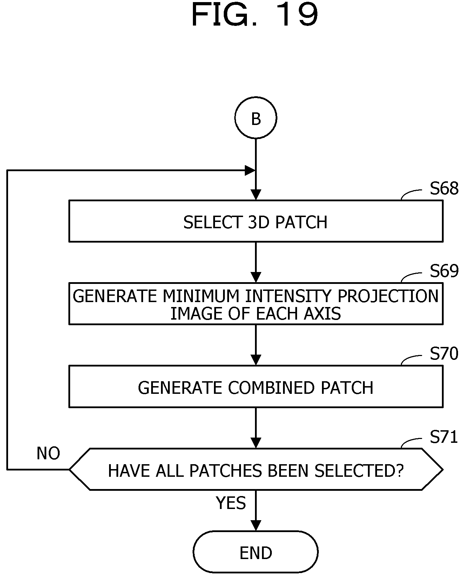

FIGs. 17 to 19 are exemplary flowcharts illustrating a procedure of the combined 2D patch generation process. The processes in FIGs. 17 to 19 correspond to the individual processing of steps S45 to S48 in FIG. 16. For example, the processes in FIGs. 17 to 19 are performed for each of the internal normal 3D patch, the boundary normal 3D patch, the internal tumor 3D patch, and the boundary tumor 3D patch.

[Step S51] The patch center point candidate extraction unit 124 selects one voxel of the organ region in the volume data.

[Step S52] The patch center point candidate extraction unit 124 determines whether the distance value of the selected voxel in the first distance image satisfies a condition C1. In step S45, the condition C1a described above is used as the condition C1. In step S46, the condition C1b described above is used as the condition C1. In step S47, the condition C1c described above is used as the condition C1. In step S48, the condition C1d described above is used as the condition C1. The process proceeds to step S53 if the condition C1 is satisfied, and the process proceeds to step S55 if the condition C1 is not satisfied.

[Step S53] The patch center point candidate extraction unit 124 determines whether the distance value of the selected voxel in the second distance image satisfies a condition C2. In step S45, the condition C2a described above is used as the condition C2. In step S46, the condition C2b described above is used as the condition C2. In step S47, the condition C2c described above is used as the condition C2. In step S48, the condition C2d described above is used as the condition C2. The process proceeds to step S54 if the condition C2 is satisfied, and the process proceeds to step S55 if the condition C2 is not satisfied.

[Step S54] The patch center point candidate extraction unit 124 selects the voxel selected in step S51 as a center point candidate of the applicable 3D patch. The "applicable 3D patch" indicates the internal normal 3D patch in step S45, the boundary normal 3D patch in step S46, the internal tumor 3D patch in step S47, and the boundary tumor 3D patch in step S48.

[Step S55] The patch center point candidate extraction unit 124 determines whether all voxels of the organ region in the volume data have been selected in step S51. If there is an unselected voxel, the process proceeds to step S51 to select one unselected voxel. On the other hand, if all the voxels have been selected, the process proceeds to step S56.

[Step S56] The patch center point candidate extraction unit 124 randomly selects N1 candidates from the selected center point candidates. This narrows down the center point candidates. In the following processing, the narrowed-down N1 center points are to be processed.

[Step S57] The patch center point determination unit 125 selects one of the center point candidates.

[Step S58] The patch center point determination unit 125 cuts out a 3D patch centered on the selected center point from the volume data. Note that a voxel of the 3D patch not included in the volume data may be interpolated based on a plurality of adjacent voxels, or may be treated as having no data.

[Step S59] The patch center point determination unit 125 calculates an average luminance value of each voxel in the cut out 3D patch and a luminance standard deviation of each voxel.

[Step S60] The patch center point determination unit 125 determines whether all the center point candidates have been selected in step S57. If there is an unselected center point candidate, the process proceeds to step S57 to select the unselected center point candidate. On the other hand, if all the center point candidates have been selected, the process proceeds to step S61.

Subsequently, a process of narrowing down the N1 center point candidates in such a manner that the luminance distribution varies among the 3D patches corresponding to the center points is performed. The following process of steps S61 to S67 is an example of such a narrowing-down processing procedure.

[Step S61] The patch center point determination unit 125 generates a two-dimensional histogram based on the average luminance value calculated in step S58 and the luminance standard deviation calculated in step S59. In this two-dimensional histogram, a frequency is counted in units of a predetermined width for each of the average value and the standard deviation. For example, a width of the average value is set to "20" and a width of the standard deviation is set to "5".

[Step S62] The patch center point determination unit 125 sets the number of blocks N3. For example, if the luminance varies from "100" to "220", the luminance is divided into sixths by $(220 - 100)/20 = 6$. Furthermore, if the standard deviation varies from "5" to "50", the standard deviation is divided into ninths by $(50 - 5)/5 = 9$. In this case, the number of divided blocks N3 is set to $6 \times 9 = 45$.

The patch center point determination unit 125 sets the initial number of patches N4 in each block by calculating N2/N3.

[Step S63] The patch center point determination unit 125 sorts the combinations of the average luminance value and the luminance standard deviation in the two-dimensional histogram in ascending order of frequency.

[Step S64] The patch center point determination unit 125 divides the sorted combinations described above into N3 blocks, and selects one block in ascending order of frequency.

[Step S65] The patch center point determination unit 125 randomly extracts up to N4 3D patches from among the 3D patches corresponding to the center point candidates included in the block.

[Step S66] The number of 3D patches extracted in step S65 is set as N5. The patch center point determination unit 125 updates the current N3 to (N3 - 1), and updates the initial number of patches N4 by calculating (N1 - N5)/N3 using the updated N3, N1, and N5.

[Step S67] The patch center point determination unit 125 determines whether all the divided blocks have been selected in step S64. If there is an unselected block, the process proceeds to step S64 to select the unselected block.

On the other hand, if all the blocks have been selected, the process proceeds to step S68. In this case, the 3D patches extracted in step S65 are the 3D patches based on the center points narrowed down from the center point candidates. In step S45, the internal normal 3D patch is determined. In step S46, the boundary normal 3D patch is determined. In step S47, the internal tumor 3D patch is determined. In step S48, the boundary tumor 3D patch is determined. Those 3D patches are to be processed in the following process.

[Step S68] The minimum intensity projection image generation unit 126 selects one 3D patch.

[Step S69] The minimum intensity projection image generation unit 126 generates a minimum intensity projection image for each of the three axial directions based on the selected 3D patch.

[Step S70] The combined patch generation unit 127 combines the individual generated minimum intensity projection images by the method illustrated in FIG. 7 to generate a combined 2D patch. The combined patch generation unit 127 adds a label to the generated combined 2D patch. A label indicating normal is added in steps S45 and S46, and a label indicating a tumor is added in steps S47 and S48.

[Step S71] The combined patch generation unit 127 determines whether all the applicable 3D patches have been selected in step S68. If there is an unselected 3D patch, the process proceeds to step S68 to select the unselected 3D patch. On the other hand, if all the 3D patches have been selected, the combined 2D patch generation process is terminated. In this case, the process proceeds to step S49 in FIG. 16, and the training of the classifier using the generated labeled combined 2D patch is carried out.

Next, processing of the image identification device 22 will be described.

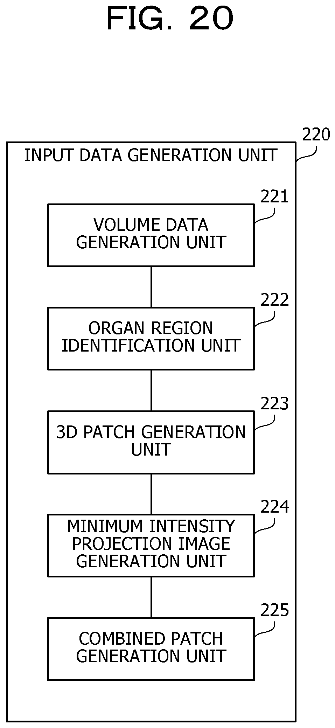

FIG. 20 is a diagram illustrating an exemplary internal configuration of the input data generation unit. As illustrated in FIG. 20, the input data generation unit 220 includes a volume data generation unit 221, an organ region identification unit 222, a 3D patch generation unit 223, a minimum intensity projection image generation unit 224, and a combined patch generation unit 225.

The volume data generation unit 221 generates three-dimensional volume data based on the tomographic image set obtained by the imaging using the MRI apparatus 21.

The organ region identification unit 222 identifies an organ region (liver region in this case) from the volume data using the classifier for organ region identification based on the trained model 211.

The 3D patch generation unit 223 selects a 3D patch center point from the organ region in the volume data, and generates a 3D patch centered on the selected center point.

The minimum intensity projection image generation unit 224 generates minimum intensity projection images in the three axial directions for each of the 3D patches corresponding to the individual determined center points.

For each of the 3D patches, the combined patch generation unit 225 combines the generated minimum intensity projection images to generate a combined 2D patch, and inputs it to the tumor identification unit 230.

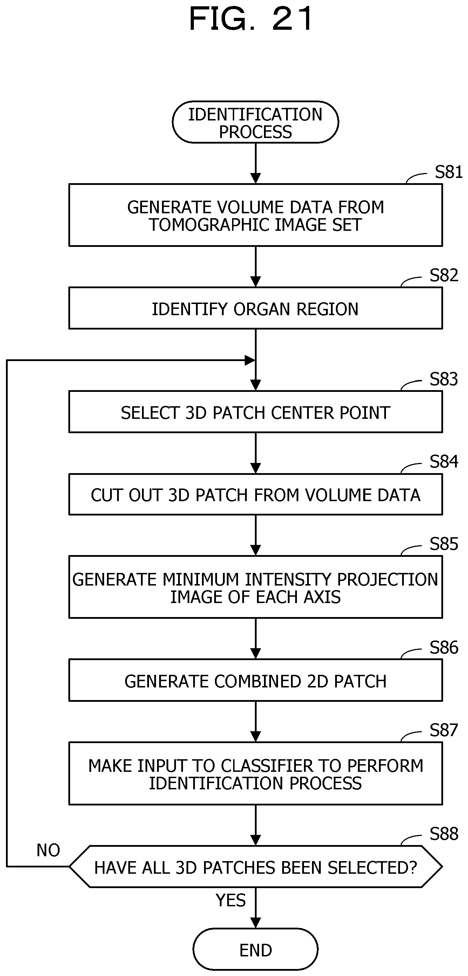

FIG. 21 is an exemplary flowchart illustrating an identification processing procedure of the image identification device.

[Step S81] The volume data generation unit 221 obtains a tomographic image set from the MRI apparatus 21. The volume data generation unit 221 generates three-dimensional volume data based on the obtained tomographic image set.

[Step S82] The organ region identification unit 222 identifies an organ region from the volume data using the classifier for organ region identification based on the trained model 211.

This trained model 211 is generated by, for example, deep learning using a large number of tomographic images as teacher images and a teacher label indicating whether each pixel in the tomographic image is an intra-organ region or an extra-organ region. In this case, in step S82, each tomographic image included in the tomographic image set is input to such a classifier based on the trained model 211 to determine whether each pixel in the individual tomographic images is the intra-organ region or the extra-organ region. Then, based on the determination result for each tomographic image, voxels in the organ region are identified among the voxels in the volume data.

[Step S83] The 3D patch generation unit 223 selects the 3D patch center point from the voxels in the organ region. Here, a 3D patch is generated by, for example, the volume data being divided into the same size. Alternatively, a 3D patch may be generated by a three-dimensional window of the same size as the 3D patch being moved for a predetermined distance (e.g., half the patch size). In step S83, one of the center points of the 3D patches generated in this manner is selected. Note that the size of the 3D patch is the same as that of the 3D patch generated during the training process.

[Step S84] The 3D patch generation unit 223 cuts out the 3D patch centered on the selected center point from the volume data. For a value of a voxel not included in the volume data among the 3D patch voxels, for example, the value of the nearest voxel is diverted or interpolation is carried out using values of multiple nearby voxels.

[Step S85] The minimum intensity projection image generation unit 224 generates a minimum intensity projection image for each of the three axial directions based on the cut out 3D patch.

[Step S86] The combined patch generation unit 225 combines the individual generated minimum intensity projection images by the method illustrated in FIG. 7 to generate a combined 2D patch.

[Step S87] The tumor identification unit 230 inputs the generated combined 2D patch to the classifier based on the trained model 100, and identifies whether the 3D patch corresponding to the combined 2D patch is a tumor or normal.

[Step S88] The tumor identification unit 230 determines whether all the generated 3D patch center points have been selected in step S83. If there is an unselected center point, the process proceeds to step S83 to select the unselected center point as a 3D patch center point. On the other hand, if all the applicable center points have been selected, the identification process is terminated. In this case, for example, identification result information in which information indicating a tumor or normal is added to each 3D patch is output as an identification result.

According to the process described above, the lesion identification is carried out using minimum intensity projection images in multiple directions based on 3D patches, whereby it becomes possible to highly accurately distinguish between a tumor or normal for each 3D patch. For example, it becomes possible to lower the possibility that a blood vessel region is erroneously identified as a tumor. Furthermore, since 2D data is input to the classifier instead of 3D data, it becomes possible to shorten the time needed for the identification process, and to reduce the memory capacity needed for the identification process as compared to the case of using the 3D data.

Note that the processing functions of the device (e.g., image identification device 1, training processing device 12, and image identification device 22) indicated in the individual embodiments described above may be implemented by a computer. In that case, a program describing the processing content of the functions to be held by each device is provided, and the processing functions described above are implemented on the computer by execution of the program on the computer. The program describing the processing content may be recorded in a computer-readable recording medium. Examples of the computer-readable recording medium include a magnetic storage device, an optical disk, a semiconductor memory, and the like. Examples of the magnetic storage device include a hard disk drive (HDD), a magnetic tape, and the like. Examples of the optical disk include a compact disc (CD), a digital versatile disc (DVD), a Blu-ray disc (BD, registered trademark), and the like.

In a case where the program is to be distributed, for example, portable recording media such as DVDs and CDs in which the program is recorded are sold. Furthermore, it is also possible to store the program in a storage device of a server computer and transfer the program from the server computer to another computer via a network.

The computer that executes the program stores, for example, the program recorded in the portable recording medium or the program transferred from the server computer in its own storage device. Then, the computer reads the program from its own storage device, and executes processing according to the program. Note that the computer may read the program directly from the portable recording medium and execute the processing according to the program. Furthermore, the computer may sequentially execute processing according to the received program each time the program is transferred from the server computer coupled via the network.

**Claims**

1.    An image identification program that causes at least one computer to execute a process, the process comprising:

acquiring a three-dimensional partial area (3) of a certain size from three-dimensional volume data (2) generated based on a plurality of tomographic images obtained by imaging an inside of a human body;
generating a plurality of projection images (4a-c) by performing extreme intensity projection on each voxel value of the partial area (3) in a plurality of mutually orthogonal directions, the extreme intensity projection being one intensity projection selected from minimum intensity projection and maximum intensity projection; and
specifying one state of the partial area (3) among a plurality of states based on the plurality of projection images (4a-c), wherein
the specifying includes using a trained model (100) generated by machine learning, and
wherein the process further comprises generating the trained model (100) by:

acquiring a plurality of training partial areas each of which is a three-dimensional area of the certain size from three-dimensional training volume data based on a plurality of training tomographic images obtained by imaging the inside of the human body;
extracting, from the plurality of training partial areas, a plurality of first partial areas that corresponds to a first state among the plurality of states and a plurality of second partial areas that corresponds to a second state among the plurality of states;
generating, for each of the plurality of first partial areas, a first projection image group that includes a plurality of first projection images generated by performing the minimum intensity projection or the maximum intensity projection on each voxel value of the corresponding first partial area in the plurality of directions;
generating, for each of the plurality of second partial areas, a second projection image group that includes a plurality of second projection images generated by performing extreme intensity projection on each voxel value of the corresponding second partial area in the plurality of directions;
executing machine learning by using the first projection image group that corresponds to each of the plurality of first partial areas as training data that corresponds to the first state and using the second projection image group that corresponds to each of the plurality of second partial areas as training data that corresponds to the second state;
extracting a plurality of first partial area candidates that corresponds to the first state and a plurality of second partial area candidates that corresponds to the second state from the plurality of training partial areas;
extracting the plurality of first partial areas from the plurality of first partial area candidates such that luminance distribution in the first partial area candidates is distributed among the first partial area candidates; and
extracting the plurality of second partial areas from the plurality of second partial area candidates such that the luminance distribution in the second partial area candidates is distributed among the second partial area candidates.

2.    The image identification program according to claim 1, wherein the process further comprises

generating a two-dimensional combined image by combining the plurality of projection images (4a-c), and the specifying includes specifying one state of the partial area (3) among the plurality of states based on the combined image.

3. An image identification device (1) comprising:
a control unit (1a) configured to:

acquire a three-dimensional partial area (3) of a certain size from three-dimensional volume data (2) generated based on a plurality of tomographic images obtained by imaging an inside of a human body,

generate a plurality of projection images (4a-c) by performing extreme intensity projection on each voxel value of the partial area (3) in a plurality of mutually orthogonal directions, the extreme intensity projection being one intensity projection selected from minimum intensity projection and maximum intensity projection, and

specify one state of the partial area (3) among a plurality of states based on the plurality of projection images (4a-c), wherein

the specifying includes using a trained model (100) generated by machine learning, and

wherein the control unit (1a) is further configured to generate the trained model (100) by:

acquiring a plurality of training partial areas each of which is a three-dimensional area of the certain size from three-dimensional training volume data based on a plurality of training tomographic images obtained by imaging the inside of the human body;

extracting, from the plurality of training partial areas, a plurality of first partial areas that corresponds to a first state among the plurality of states and a plurality of second partial areas that corresponds to a second state among the plurality of states;

generating, for each of the plurality of first partial areas, a first projection image group that includes a plurality of first projection images generated by performing the minimum intensity projection or the maximum intensity projection on each voxel value of the corresponding first partial area in the plurality of directions;

generating, for each of the plurality of second partial areas, a second projection image group that includes a plurality of second projection images generated by performing extreme intensity projection on each voxel value of the corresponding second partial area in the plurality of directions;

executing machine learning by using the first projection image group that corresponds to each of the plurality of first partial areas as training data that corresponds to the first state and using the second projection image group that corresponds to each of the plurality of second partial areas as training data that corresponds to the second state;

extracting a plurality of first partial area candidates that corresponds to the first state and a plurality of second partial area candidates that corresponds to the second state from the plurality of training partial areas;

extracting the plurality of first partial areas from the plurality of first partial area candidates such that luminance distribution in the first partial area candidates is distributed among the first partial area candidates; and

extracting the plurality of second partial areas from the plurality of second partial area candidates such that the luminance distribution in the second partial area candidates is distributed among the second partial area candidates.

4. An image identification method for a computer to execute a process comprising:

acquiring a three-dimensional partial area (3) of a certain size from three-dimensional volume data (2) generated based on a plurality of tomographic images obtained by imaging an inside of a human body;

generating a plurality of projection images (4a-c) by performing extreme intensity projection on each voxel value of the partial area (3) in a plurality of mutually orthogonal directions, the extreme intensity projection being one intensity projection selected from minimum intensity projection and maximum intensity projection; and

specifying one state of the partial area (3) among a plurality of states based on the plurality of projection images (4a-c), wherein

the specifying includes using a trained model (100) generated by machine learning, and

wherein the process further comprises generating the trained model (100) by:

acquiring a plurality of training partial areas each of which is a three-dimensional area of the certain size from three-dimensional training volume data based on a plurality of training tomographic images obtained by imaging the inside of the human body;

extracting, from the plurality of training partial areas, a plurality of first partial areas that corresponds to a first state among the plurality of states and a plurality of second partial areas that corresponds to a second state among the plurality of states;

generating, for each of the plurality of first partial areas, a first projection image group that includes a plurality of first projection images generated by performing the minimum intensity projection or the maximum intensity projection on each voxel value of the corresponding first partial area in the plurality of directions;

generating, for each of the plurality of second partial areas, a second projection image group that includes a plurality of second projection images generated by performing extreme intensity projection on each voxel value of the corresponding second partial area in the plurality of directions;

executing machine learning by using the first projection image group that corresponds to each of the plurality of first partial areas as training data that corresponds to the first state and using the second projection image group that corresponds to each of the plurality of second partial areas as training data that corresponds to the second state;

extracting a plurality of first partial area candidates that corresponds to the first state and a plurality of second partial area candidates that corresponds to the second state from the plurality of training partial areas;

extracting the plurality of first partial areas from the plurality of first partial area candidates such that luminance distribution in the first partial area candidates is distributed among the first partial area candidates; and

extracting the plurality of second partial areas from the plurality of second partial area candidates such that the luminance distribution in the second partial area candidates is distributed among the second partial area candidates.

5. The image identification method according to claim 4, wherein the process further comprises

generating a two-dimensional combined image by combining the plurality of projection images (4a-c), and
the specifying includes specifying one state of the partial area (3) among the plurality of states based on the combined image.

## Patentansprüche

1. Bildidentifizierungsprogramm, das mindestens einen Computer veranlasst, einen Prozess auszuführen, wobei der Prozess Folgendes umfasst:

Erfassen eines dreidimensionalen Teilbereichs (3) einer bestimmten Größe aus dreidimensionalen Volumendaten (2), die basierend auf einer Vielzahl von tomographischen Bildern erzeugt werden, die durch Bildgebung des Inneren eines menschlichen Körpers erhalten werden;

Erzeugen einer Vielzahl von Projektionsbildern (4a-c) durch Durchführen einer extremen Intensitätsprojektion auf jeden Voxelwert des Teilbereichs (3) in einer Vielzahl von zueinander orthogonalen Richtungen, wobei die extreme Intensitätsprojektion eine Intensitätsprojektion ist, die aus einer minimalen Intensitätsprojektion und einer maximalen Intensitätsprojektion ausgewählt ist; und

Spezifizieren eines Zustands des Teilbereichs (3) unter einer Vielzahl von Zuständen basierend auf der Vielzahl von Projektionsbildern (4a-c), wobei

das Spezifizieren Verwenden eines trainierten Modells (100) einschließt, das durch maschinelles Lernen erzeugt wird, und

wobei der Prozess weiter Erzeugen des trainierten Modells (100) umfasst durch:

Erfassen einer Vielzahl von Trainingsteilbereichen, von denen jeder ein dreidimensionaler Bereich einer bestimmten Größe ist, aus dreidimensionalen Trainingsvolumendaten basierend auf einer Vielzahl von Trainingstomographiebildern, die durch Bildgebung des Inneren des menschlichen Körpers erhalten werden;

Extrahieren, aus der Vielzahl von Trainingsteilbereichen, einer Vielzahl von ersten Teilbereichen, die einem ersten Zustand aus der Vielzahl von Zuständen entsprechen, und einer Vielzahl von zweiten Teilbereichen, die einem zweiten Zustand aus der Vielzahl von Zuständen entsprechen;

Erzeugen, für jeden der Vielzahl von ersten Teilbereichen, einer ersten Projektionsbildgruppe, die eine Vielzahl von ersten Projektionsbildern einschließt, die durch Durchführen der minimalen Intensitätsprojektion oder der maximalen Intensitätsprojektion auf jeden Voxelwert des entsprechenden ersten Teilbereichs in der Vielzahl von Richtungen erzeugt wird;

Erzeugen, für jeden der Vielzahl von zweiten Teilbereichen, einer zweiten Projektionsbildgruppe, die eine Vielzahl von zweiten Projektionsbildern einschließt, die durch Durchführen einer extremen Intensitätsprojektion auf jeden Voxelwert des entsprechenden zweiten Teilbereichs in der Vielzahl von Richtungen erzeugt werden;

Ausführen von maschinellem Lernen durch Verwenden der ersten Projektionsbildgruppe, die jeder der Vielzahl von ersten Teilbereichen entspricht, als Trainingsdaten, die dem ersten Zustand entsprechen, und Verwenden der zweiten Projektionsbildgruppe, die jedem der Vielzahl von zweiten Teilbereichen entspricht, als Trainingsdaten, die dem zweiten Zustand entsprechen;

Extrahieren einer Vielzahl von ersten Teilbereichskandidaten, die dem ersten Zustand entsprechen, und einer Vielzahl von zweiten Teilbereichskandidaten, die dem zweiten Zustand entsprechen, aus der Vielzahl von Trainingsteilbereichen;

Extrahieren der Vielzahl von ersten Teilbereichen aus der Vielzahl von ersten Teilbereichskandidaten, so dass eine Luminanzverteilung in den ersten Teilbereichskandidaten auf die ersten Teilbereichskandidaten verteilt wird; und

Extrahieren der Vielzahl von zweiten Teilbereichen aus der Vielzahl von zweiten Teilbereichskandidaten, so dass die Luminanzverteilung in den zweiten Teilbereichskandidaten auf die zweiten Teilbereichskandidaten verteilt ist.

2. Bildidentifizierungsprogramm nach Anspruch 1, wobei der Prozess weiter umfasst

Erzeugen eines zweidimensionalen kombinierten Bildes durch Kombinieren der Vielzahl von Projektionsbildern (4a-c), und

das Spezifizieren, das Spezifizieren eines Zustands des Teilbereichs (3) aus der Vielzahl von Zuständen basierend auf dem kombinierten Bild einschließt.

3. Bildidentifizierungsvorrichtung (1), umfassend:
eine Steuereinheit (1a), die konfiguriert ist zum:

Erfassen eines dreidimensionalen Teilbereichs (3) einer bestimmten Größe aus dreidimensionalen Volumendaten (2), die basierend auf einer Vielzahl von tomographischen Bildern erzeugt werden, die durch Bildgebung des Inneren eines menschlichen Körpers erhalten werden,

Erzeugen einer Vielzahl von Projektionsbildern (4a-c) durch Durchführen einer extremen Intensitätsprojektion auf jeden Voxelwert des Teilbereichs (3) in einer Vielzahl von zueinander orthogonalen Richtungen, wobei die extreme Intensitätsprojektion eine Intensitätsprojektion ist, die aus einer minimalen Intensitätsprojektion und einer maximalen Intensitätsprojektion ausgewählt wird, und

Spezifizieren eines Zustands des Teilbereichs (3) unter einer Vielzahl von Zuständen basierend auf der Vielzahl von Projektionsbildern (4a-c), wobei

das Spezifizieren Verwenden eines trainierten Modells (100) einschließt, das durch maschinelles Lernen erzeugt wird, und

wobei die Steuereinheit (1a) weiter konfiguriert ist, um das trainierte Modell (100) zu erzeugen durch:

Erfassen einer Vielzahl von Trainingsteilbereichen, von denen jeder ein dreidimensionaler Bereich einer bestimmten Größe ist, aus dreidimensionalen Trainingsvolumendaten basierend auf einer Vielzahl von Trainingstomographiebildern, die durch Bildgebung des Inneren des menschlichen Körpers erhalten werden;

Extrahieren, aus der Vielzahl von Trainingsteilbereichen, einer Vielzahl von ersten Teilbereichen, die einem ersten Zustand aus der Vielzahl von Zuständen entsprechen, und einer Vielzahl von zweiten Teilbereichen, die einem zweiten Zustand aus der Vielzahl von Zuständen entsprechen;

Erzeugen, für jeden der Vielzahl von ersten Teilbereichen, einer ersten Projektionsbildgruppe, die eine Vielzahl von ersten Projektionsbildern einschließt, die durch Durchführen der minimalen Intensitätsprojektion oder der maximalen Intensitätsprojektion auf jeden Voxelwert des entsprechenden ersten Teilbereichs in der Vielzahl von Richtungen erzeugt wird;

Erzeugen, für jeden der Vielzahl von zweiten Teilbereichen, einer zweiten Projektionsbildgruppe, die eine Vielzahl von zweiten Projektionsbildern einschließt, die durch Durchführen einer extremen Intensitätsprojektion auf jeden Voxelwert des entsprechenden zweiten Teilbereichs in der Vielzahl von Richtungen erzeugt werden;

Ausführen von maschinellem Lernen durch Verwenden der ersten Projektionsbildgruppe, die jeder der Vielzahl von ersten Teilbereichen entspricht, als Trainingsdaten, die dem ersten Zustand entsprechen, und Verwenden der zweiten Projektionsbildgruppe, die jedem der Vielzahl von zweiten Teilbereichen entspricht, als Trainingsdaten, die dem zweiten Zustand entsprechen;

Extrahieren einer Vielzahl von ersten Teilbereichskandidaten, die dem ersten Zustand entsprechen, und einer Vielzahl von zweiten Teilbereichskandidaten, die dem zweiten Zustand entsprechen, aus der Vielzahl von Trainingsteilbereichen;

Extrahieren der Vielzahl von ersten Teilbereichen aus der Vielzahl von ersten Teilbereichskandidaten, so dass eine Luminanzverteilung in den ersten Teilbereichskandidaten auf die ersten Teilbereichskandidaten verteilt wird; und

Extrahieren der Vielzahl von zweiten Teilbereichen aus der Vielzahl von zweiten Teilbereichskandidaten, so dass die Luminanzverteilung in den zweiten Teilbereichskandidaten auf die zweiten Teilbereichskandidaten verteilt ist.

4. Bildidentifizierungsverfahren für einen Computer zum Ausführen eines Prozesses, umfassend:

Erfassen eines dreidimensionalen Teilbereichs (3) einer bestimmten Größe aus dreidimensionalen Volumendaten (2), die basierend auf einer Vielzahl von tomographischen Bildern erzeugt werden, die durch Bildgebung des Inneren eines menschlichen Körpers erhalten werden;

Erzeugen einer Vielzahl von Projektionsbildern (4a-c) durch Durchführen einer extremen Intensitätsprojektion auf jeden Voxelwert des Teilbereichs (3) in einer Vielzahl von zueinander orthogonalen Richtungen, wobei die extreme Intensitätsprojektion eine Intensitätsprojektion ist, die aus einer minimalen Intensitätsprojektion und einer maximalen Intensitätsprojektion ausgewählt ist; und

Spezifizieren eines Zustands des Teilbereichs (3) unter einer Vielzahl von Zuständen basierend auf der Vielzahl von Projektionsbildern (4a-c), wobei

das Spezifizieren Verwenden eines trainierten Modells (100) einschließt, das durch maschinelles Lernen erzeugt wird, und

wobei der Prozess weiter Erzeugen des trainierten Modells (100) umfasst durch:

Erfassen einer Vielzahl von Trainingsteilbereichen, von denen jeder ein dreidimensionaler Bereich einer bestimmten Größe ist, aus dreidimensionalen Trainingsvolumendaten basierend auf einer Vielzahl von Trainingstomographiebildern, die durch Bildgebung des Inneren des menschlichen Körpers erhalten werden;

Extrahieren, aus der Vielzahl von Trainingsteilbereichen, einer Vielzahl von ersten Teilbereichen, die einem ersten Zustand aus der Vielzahl von Zuständen entsprechen, und einer Vielzahl von zweiten Teilbereichen, die einem zweiten Zustand aus der Vielzahl von Zuständen entsprechen;

Erzeugen, für jeden der Vielzahl von ersten Teilbereichen, einer ersten Projektionsbildgruppe, die eine Vielzahl von ersten Projektionsbildern einschließt, die durch Durchführen der minimalen Intensitätsprojektion oder der maximalen Intensitätsprojektion auf jeden Voxelwert des entsprechenden ersten Teilbereichs in der Vielzahl von Richtungen erzeugt wird;

Erzeugen, für jeden der Vielzahl von zweiten Teilbereichen, einer zweiten Projektionsbildgruppe, die eine Vielzahl von zweiten Projektionsbildern einschließt, die durch Durchführen einer extremen Intensitätsprojektion auf jeden Voxelwert des entsprechenden zweiten Teilbereichs in der Vielzahl von Richtungen erzeugt werden;

Ausführen von maschinellem Lernen durch Verwenden der ersten Projektionsbildgruppe, die jeder der Vielzahl von ersten Teilbereichen entspricht, als Trainingsdaten, die dem ersten Zustand entsprechen, und Verwenden der zweiten Projektionsbildgruppe, die jedem der Vielzahl von zweiten Teilbereichen entspricht, als Trainingsdaten, die dem zweiten Zustand entsprechen;

Extrahieren einer Vielzahl von ersten Teilbereichskandidaten, die dem ersten Zustand entsprechen, und einer Vielzahl von zweiten Teilbereichskandidaten, die dem zweiten Zustand entsprechen, aus der Vielzahl von Trainingsteilbereichen;

Extrahieren der Vielzahl von ersten Teilbereichen aus der Vielzahl von ersten Teilbereichskandidaten, so dass eine Luminanzverteilung in den ersten Teilbereichskandidaten auf die ersten Teilbereichskandidaten verteilt wird; und

Extrahieren der Vielzahl von zweiten Teilbereichen aus der Vielzahl von zweiten Teilbereichskandidaten, so dass die Luminanzverteilung in den zweiten Teilbereichskandidaten auf die zweiten Teilbereichskandidaten verteilt ist.

5. Bildidentifizierungsverfahren nach Anspruch 4, wobei der Prozess weiter umfasst

Erzeugen eines zweidimensionalen kombinierten Bildes durch Kombinieren der Vielzahl von Projektionsbildern (4a-c), und

das Spezifizieren, das Spezifizieren eines Zustands des Teilbereichs (3) aus der Vielzahl von Zuständen basierend auf dem kombinierten Bild einschließt.

**Revendications**

1. Programme d'identification d'image qui amène au moins un ordinateur à exécuter un procédé, le procédé comprenant :

   l'acquisition d'une zone partielle tridimensionnelle (3) d'une certaine taille à partir de données de volume tridimensionnel (2) générées sur la base d'une pluralité d'images tomographiques obtenues par l'imagerie d'un intérieur d'un corps humain ;
   la génération d'une pluralité d'images de projection (4a-c) par la mise en œuvre d'une projection d'intensité extrême sur chaque valeur de voxel de la zone partielle (3) dans une pluralité de directions mutuellement orthogonales, la projection d'intensité extrême étant une projection d'intensité sélectionnée à partir d'une projection d'intensité minimale et d'une projection d'intensité maximale ; et
   la spécification d'un état de la zone partielle (3) parmi une pluralité d'états sur la base de la pluralité d'images de projection (4a-c), dans lequel
   la spécification inclut l'utilisation d'un modèle entraîné (100) généré par un apprentissage automatique, et
   dans lequel le procédé comprend en outre la génération du modèle entraîné (100) par :

      l'acquisition d'une pluralité de zones partielles d'entraînement dont chacune est une zone tridimensionnelle de la certaine taille à partir de données de volume d'entraînement tridimensionnel sur la base d'une pluralité d'images tomographiques d'entraînement obtenues par l'imagerie de l'intérieur du corps humain ;
      l'extraction, à partir de la pluralité de zones partielles d'entraînement, d'une pluralité de premières zones partielles qui correspond à un premier état parmi la pluralité d'états et d'une pluralité de secondes zones partielles qui correspond à un second état parmi la pluralité d'états ;
      la génération, pour chacune de la pluralité de premières zones partielles, d'un premier groupe d'images de projection qui inclut une pluralité de premières images de projection générées par la mise en œuvre de la projection d'intensité minimale ou de la projection d'intensité maximale sur chaque valeur de voxel de la première zone partielle correspondante dans la pluralité de directions ;
      la génération, pour chacune de la pluralité de secondes zones partielles, d'un second groupe d'images de projection qui inclut une pluralité de secondes images de projection générées par la mise en œuvre de la projection d'intensité extrême sur chaque valeur de voxel de la seconde zone partielle correspondante dans la pluralité de directions ;
      la mise en œuvre d'un apprentissage automatique en utilisant le premier groupe d'images de projection qui correspond à chacune de la pluralité de premières zones partielles en tant que des données d'entraînement qui correspond au premier état et en utilisant le second groupe d'images de projection qui correspond à chacune de la pluralité de secondes zones partielles en tant que des données d'entraînement qui correspond au second état ;
      l'extraction d'une pluralité de premières zones partielles candidates qui correspond au premier état et d'une pluralité de secondes zones partielles candidates qui correspond au second état à partir de la pluralité de zones partielles d'entraînement ;
      l'extraction de la pluralité de premières zones partielles à partir de la pluralité de premières zones partielles candidates de sorte qu'une distribution de luminance dans les premières zones partielles candidates soit distribuée parmi les premières zones partielles candidates ; et
      l'extraction de la pluralité de secondes zones partielles à partir de la pluralité de secondes zones partielles candidates de sorte que la distribution de luminance dans les secondes zones partielles candidates soit distribuée parmi les secondes zones partielles candidates.

2. Programme d'identification d'image selon la revendication 1, dans lequel le procédé comprend en outre

   la génération d'une image combinée bidimensionnelle par la combinaison de la pluralité d'images de projection (4a-c), et
   la spécification inclut la spécification d'un état de la zone partielle (3) parmi la pluralité d'états sur la base de l'image combinée.

**3.** Dispositif d'identification d'image (1) comprenant :
une unité de commande (1a) configurée pour :

acquérir une zone partielle tridimensionnelle (3) d'une certaine taille à partir de données de volume tridimensionnel (2) générées sur la base d'une pluralité d'images tomographiques obtenues par l'imagerie d'un intérieur d'un corps humain,
générer une pluralité d'images de projection (4a-c) par la mise en œuvre d'une projection d'intensité extrême sur chaque valeur de voxel de la zone partielle (3) dans une pluralité de directions mutuellement orthogonales, la projection d'intensité extrême étant une projection d'intensité sélectionnée à partir d'une projection d'intensité minimale et d'une projection d'intensité maximale, et
spécifier un état de la zone partielle (3) parmi une pluralité d'états sur la base de la pluralité d'images de projection (4a-c), dans lequel
la spécification inclut l'utilisation d'un modèle entraîné (100) généré par un apprentissage automatique, et
dans lequel l'unité de commande (1a) est en outre configurée pour générer le modèle entraîné (100) par :

l'acquisition d'une pluralité de zones partielles d'entraînement dont chacune est une zone tridimensionnelle de la certaine taille à partir de données de volume d'entraînement tridimensionnel sur la base d'une pluralité d'images tomographiques d'entraînement obtenues par l'imagerie de l'intérieur du corps humain ;
l'extraction, à partir de la pluralité de zones partielles d'entraînement, d'une pluralité de premières zones partielles qui correspond à un premier état parmi la pluralité d'états et d'une pluralité de secondes zones partielles qui correspond à un second état parmi la pluralité d'états ;
la génération, pour chacune de la pluralité de premières zones partielles, d'un premier groupe d'images de projection qui inclut une pluralité de premières images de projection générées par la mise en œuvre de la projection d'intensité minimale ou de la projection d'intensité maximale sur chaque valeur de voxel de la première zone partielle correspondante dans la pluralité de directions ;
la génération, pour chacune de la pluralité de secondes zones partielles, d'un second groupe d'images de projection qui inclut une pluralité de secondes images de projection générées par la mise en œuvre de la projection d'intensité extrême sur chaque valeur de voxel de la seconde zone partielle correspondante dans la pluralité de directions ;
la mise en œuvre d'un apprentissage automatique en utilisant le premier groupe d'images de projection qui correspond à chacune de la pluralité de premières zones partielles en tant que des données d'entraînement qui correspond au premier état et en utilisant le second groupe d'images de projection qui correspond à chacune de la pluralité de secondes zones partielles en tant que des données d'entraînement qui correspond au second état ;
l'extraction d'une pluralité de premières zones partielles candidates qui correspond au premier état et d'une pluralité de secondes zones partielles candidates qui correspond au second état à partir de la pluralité de zones partielles d'entraînement ;
l'extraction de la pluralité de premières zones partielles à partir de la pluralité de premières zones partielles candidates de sorte qu'une distribution de luminance dans les premières zones partielles candidates soit distribuée parmi les premières zones partielles candidates ; et
l'extraction de la pluralité de secondes zones partielles à partir de la pluralité de secondes zones partielles candidates de sorte que la distribution de luminance dans les secondes zones partielles candidates soit distribuée parmi les secondes zones partielles candidates.

**4.** Procédé d'identification d'image pour qu'un ordinateur exécute un procédé comprenant :

l'acquisition d'une zone partielle tridimensionnelle (3) d'une certaine taille à partir de données de volume tridimensionnel (2) générées sur la base d'une pluralité d'images tomographiques obtenues par l'imagerie d'un intérieur d'un corps humain ;
la génération d'une pluralité d'images de projection (4a-c) par la mise en œuvre d'une projection d'intensité extrême sur chaque valeur de voxel de la zone partielle (3) dans une pluralité de directions mutuellement orthogonales, la projection d'intensité extrême étant une projection d'intensité sélectionnée à partir d'une projection d'intensité minimale et d'une projection d'intensité maximale ; et
la spécification d'un état de la zone partielle (3) parmi une pluralité d'états sur la base de la pluralité d'images de projection (4a-c), dans lequel
la spécification inclut l'utilisation d'un modèle entraîné (100) généré par un apprentissage automatique, et
dans lequel le procédé comprend en outre la génération du modèle entraîné (100) par :

l'acquisition d'une pluralité de zones partielles d'entraînement dont chacune est une zone tridimensionnelle de la certaine taille à partir de données de volume d'entraînement tridimensionnel sur la base d'une pluralité d'images tomographiques d'entraînement obtenues par l'imagerie de l'intérieur du corps humain ;

l'extraction, à partir de la pluralité de zones partielles d'entraînement, d'une pluralité de premières zones partielles qui correspond à un premier état parmi la pluralité d'états et d'une pluralité de secondes zones partielles qui correspond à un second état parmi la pluralité d'états ;

la génération, pour chacune de la pluralité de premières zones partielles, d'un premier groupe d'images de projection qui inclut une pluralité de premières images de projection générées par la mise en œuvre de la projection d'intensité minimale ou de la projection d'intensité maximale sur chaque valeur de voxel de la première zone partielle correspondante dans la pluralité de directions ;

la génération, pour chacune de la pluralité de secondes zones partielles, d'un second groupe d'images de projection qui inclut une pluralité de secondes images de projection générées par la mise en œuvre de la projection d'intensité extrême sur chaque valeur de voxel de la seconde zone partielle correspondante dans la pluralité de directions ;

la mise en œuvre d'un apprentissage automatique en utilisant le premier groupe d'images de projection qui correspond à chacune de la pluralité de premières zones partielles en tant que des données d'entraînement qui correspond au premier état et en utilisant le second groupe d'images de projection qui correspond à chacune de la pluralité de secondes zones partielles en tant que des données d'entraînement qui correspond au second état ;

l'extraction d'une pluralité de premières zones partielles candidates qui correspond au premier état et d'une pluralité de secondes zones partielles candidates qui correspond au second état à partir de la pluralité de zones partielles d'entraînement ;

l'extraction de la pluralité de premières zones partielles à partir de la pluralité de premières zones partielles candidates de sorte qu'une distribution de luminance dans les premières zones partielles candidates soit distribuée parmi les premières zones partielles candidates ; et

l'extraction de la pluralité de secondes zones partielles à partir de la pluralité de secondes zones partielles candidates de sorte que la distribution de luminance dans les secondes zones partielles candidates soit distribuée parmi les secondes zones partielles candidates.

5. Procédé d'identification d'image selon la revendication 4, dans lequel le procédé comprend en outre

la génération d'une image combinée bidimensionnelle par la combinaison de la pluralité d'images de projection (4a-c), et

la spécification inclut la spécification d'un état de la zone partielle (3) parmi la pluralité d'états sur la base de l'image combinée.

# FIG. 1

# FIG. 2

MRI APPARATUS — 11

MRI APPARATUS — 21

TRAINING PROCESSING DEVICE — 12

TRAINED MODEL — 100

IMAGE IDENTIFICATION DEVICE — 22

# FIG. 3

# FIG. 4

OBTAIN TOMOGRAPHIC IMAGE SET    S11

GENERATE 2D PATCH    S12

IDENTIFY LESION WITH CLASSIFIER    S13

LESION
A

LESION
B

NORMAL

# FIG. 5

# FIG. 6

37c  37b  37a

TRAINING

34c  35

30

CLASSIFIER  36

# FIG. 7

# FIG. 8

TRAINING PROCESSING DEVICE — 12

TRAINING DATA GENERATION UNIT — 120

CLASSIFIER TRAINING UNIT — 130

TRAINED MODEL (FOR TUMOR IDENTIFICATION) — 110, 100

STORAGE UNIT

IMAGE IDENTIFICATION DEVICE — 22

STORAGE UNIT — 210

TRAINED MODEL (FOR ORGAN REGION IDENTIFICATION) — 211

TRAINED MODEL (FOR TUMOR IDENTIFICATION) — 100

INPUT DATA GENERATION UNIT — 220

TUMOR IDENTIFICATION UNIT — 230

# FIG. 9

120

### TRAINING DATA GENERATION UNIT

121

VOLUME DATA
GENERATION UNIT

122

ANNOTATION UNIT

123

DISTANCE IMAGE
GENERATION UNIT

124

PATCH CENTER POINT
CANDIDATE
EXTRACTION UNI

125

PATCH CENTER POINT
DETERMINATION UNIT

126

MINIMUM INTENSITY
PROJECTION IMAGE
GENERATION UNIT

127

COMBINED PATCH
GENERATION UNIT

# FIG. 10

# FIG. 11

FORWARD DIRECTION
RASTER SCANNING

REVERSE DIRECTION
RASTER SCANNING

# FIG. 12

FORWARD DIRECTION
RASTER SCANNING

REVERSE DIRECTION
RASTER SCANNING

FIG. 13

<NORMAL 3D PATCH
DETERMINATION PROCESS>

<TUMOR 3D PATCH
DETERMINATION PROCESS>

FIG. 14

| DISTANCE D1 OF FIRST DISTANCE IMAGE (DISTANCE TO ORGAN BOUNDARY) | DISTANCE D2 OF SECOND DISTANCE IMAGE (DISTANCE TO TUMOR BOUNDARY) |

PATCH CENTER POINT CANDIDATE EXTRACTION UNIT — 124

S31a
EXTRACT N1 CENTER POINTS SATISFYING FOLLOWING CONDITIONS
(CONDITION C1c)$D1(x,y,z) > (S/2)*3^{1/2}$
(CONDITION C2c)$D2(x,y,z) \leqq (S/2)*3^{1/2}$

S31b
EXTRACT N1 CENTER POINTS SATISFYING FOLLOWING CONDITIONS
(CONDITION C1d)$D1(x,y,z) \leqq (S/2)*3^{1/2}$
(CONDITION 21d)$D2(x,y,z) \leqq (S/2)*3^{1/2}$

CENTER POINT CANDIDATE OF INTERNAL TUMOR 3D PATCH

CENTER POINT CANDIDATE OF BOUNDARY TUMOR 3D PATCH

PATCH CENTER POINT DETERMINATION UNIT — 125

S32a
CALCULATE 3D PATCH LUMINANCE AVERAGE

S32b
CALCULATE 3D PATCH LUMINANCE STANDARD DEVIATION

S32c
CALCULATE 3D PATCH LUMINANCE AVERAGE

S32d
CALCULATE 3D PATCH LUMINANCE STANDARD DEVIATION

S33a
EXTRACT N2 (< N1) PIECES SUCH THAT AVERAGE AND STANDARD DEVIATION ARE DISTRIBUTED

S33b
EXTRACT N2 (< N1) PIECES SUCH THAT AVERAGE AND STANDARD DEVIATION ARE DISTRIBUTED

# FIG. 15

# FIG. 16

```
        ┌─────────────────┐
        │    TRAINING     │
        │    PROCESS      │
        └─────────────────┘
                 │
                 ▼                              ┌─ S41
┌──────────────────────────────────────┐
│    GENERATE VOLUME DATA FROM          │
│    TOMOGRAPHIC IMAGE SET              │
└──────────────────────────────────────┘
                 │
                 ▼                              ┌─ S42
┌──────────────────────────────────────┐
│  PERFORM ANNOTATION TO INTRA-ORGAN/   │
│  EXTRA-ORGAN REGION AND TUMOR REGION/ │
│           NORMAL REGION               │
└──────────────────────────────────────┘
                 │
                 ▼                              ┌─ S43
┌──────────────────────────────────────┐
│  GENERATE BINARY IMAGE REPRESENTING   │
│   ORGAN REGION AND BINARY IMAGE       │
│  REPRESENTING NORMAL REGION (NON-     │
│          TUMOR REGION)                │
└──────────────────────────────────────┘
                 │
                 ▼                              ┌─ S44
┌──────────────────────────────────────┐
│  GENERATE FIRST DISTANCE IMAGE AND    │
│     SECOND DISTANCE IMAGE             │
└──────────────────────────────────────┘
                 │
                 ▼                              ┌─ S45
┌┌────────────────────────────────────┐┐
││  COMBINED 2D PATCH GENERATION       ││
││  PROCESS BASED ON INTERNAL NORMAL   ││
││           3D PATCH                  ││
└└────────────────────────────────────┘┘
                 │
                 ▼                              ┌─ S46
┌┌────────────────────────────────────┐┐
││  COMBINED 2D PATCH GENERATION       ││
││  PROCESS BASED ON BOUNDARY NORMAL   ││
││           3D PATCH                  ││
└└────────────────────────────────────┘┘
                 │
                 ▼                              ┌─ S47
┌┌────────────────────────────────────┐┐
││  COMBINED 2D PATCH GENERATION       ││
││ PROCESS BASED ON INTERNAL TUMOR 3D  ││
││            PATCH                    ││
└└────────────────────────────────────┘┘
                 │
                 ▼                              ┌─ S48
┌┌────────────────────────────────────┐┐
││  COMBINED 2D PATCH GENERATION       ││
││  PROCESS BASED ON BOUNDARY TUMOR    ││
││           3D PATCH                  ││
└└────────────────────────────────────┘┘
                 │
                 ▼                              ┌─ S49
┌──────────────────────────────────────┐
│           TRAIN CLASSIFIER            │
└──────────────────────────────────────┘
                 │
                 ▼
        ┌─────────────────┐
        │       END       │
        └─────────────────┘
```

# FIG. 17

COMBINED 2D PATCH
GENERATION PROCESS

S51
SELECT VOXEL

S52
IS CONDITION C1 SATISFIED?　　NO

YES

S53
IS CONDITION C2 SATISFIED?　　NO

YES

S54
MAKE SELECTION AS CENTER POINT
CANDIDATE

S55
NO　　HAVE ALL VOXELS BEEN SELECTED?

YES

S56
RANDOMLY SELECT N1 CANDIDATES FROM
SELECTED CANDIDATES

S57
SELECT CENTER POINT CANDIDATE

S58
CUT OUT 3D PATCH FROM VOLUME DATA

S59
CALCULATE AVERAGE LUMINANCE VALUE
AND LUMINANCE STANDARD DEVIATION

S60
NO　　HAVE ALL CENTER POINT CANDIDATES
BEEN SELECTED?

YES

A

# FIG. 18

A

S61
GENERATE TWO-DIMENSIONAL HISTOGRAM OF AVERAGE LUMINANCE VALUE AND LUMINANCE STANDARD DEVIATION

S62
SET INITIAL NUMBER OF PATCHES N4 IN EACH BLOCK WITH RESPECT TO NUMBER OF DIVIDED BLOCKS N3 AS N4 = N2/N3

S63
SORT COMBINATIONS OF AVERAGE LUMINANCE VALUE AND LUMINANCE STANDARD DEVIATION IN ASCENDING ORDER OF FREQUENCY

S64
SELECT BLOCK

S65
EXTRACT UP TO N4 3D PATCHES FROM BLOCK

S66
$$N3 = N3 - 1$$
$$N4 = (N1 - N5)/N3$$

S67
NO ⟨ HAVE ALL BLOCKS BEEN SELECTED? ⟩

YES

B

# FIG. 19

( B )

↓

SELECT 3D PATCH — S68

↓

GENERATE MINIMUM INTENSITY PROJECTION IMAGE OF EACH AXIS — S69

↓

GENERATE COMBINED PATCH — S70

↓

NO ← HAVE ALL PATCHES BEEN SELECTED? — S71

YES ↓

( END )

# FIG. 20

```
┌─────────────────────────────────────────┐ 220
│        INPUT DATA GENERATION UNIT        │
│                                          │
│   ┌──────────────────────────────┐ 221   │
│   │        VOLUME DATA           │       │
│   │     GENERATION UNIT          │       │
│   └──────────────────────────────┘       │
│                  │                       │
│   ┌──────────────────────────────┐ 222   │
│   │       ORGAN REGION           │       │
│   │    IDENTIFICATION UNIT       │       │
│   └──────────────────────────────┘       │
│                  │                       │
│   ┌──────────────────────────────┐ 223   │
│   │     3D PATCH GENERATION      │       │
│   │            UNIT              │       │
│   └──────────────────────────────┘       │
│                  │                       │
│   ┌──────────────────────────────┐ 224   │
│   │    MINIMUM INTENSITY         │       │
│   │    PROJECTION IMAGE          │       │
│   │    GENERATION UNIT           │       │
│   └──────────────────────────────┘       │
│                  │                       │
│   ┌──────────────────────────────┐ 225   │
│   │     COMBINED PATCH           │       │
│   │     GENERATION UNIT          │       │
│   └──────────────────────────────┘       │
│                                          │
└─────────────────────────────────────────┘
```

# FIG. 21

```
          ( IDENTIFICATION )
          (    PROCESS     )
                 │
                 ▼                                    ⌐ S81
   ┌─────────────────────────────────────┐
   │     GENERATE VOLUME DATA FROM        │
   │       TOMOGRAPHIC IMAGE SET          │
   └─────────────────────────────────────┘
                 │
                 ▼                                    ⌐ S82
   ┌─────────────────────────────────────┐
   │         IDENTIFY ORGAN REGION        │
   └─────────────────────────────────────┘
                 │
    ┌───────────►│
    │            ▼                                    ⌐ S83
    │  ┌─────────────────────────────────────┐
    │  │      SELECT 3D PATCH CENTER POINT    │
    │  └─────────────────────────────────────┘
    │            │
    │            ▼                                    ⌐ S84
    │  ┌─────────────────────────────────────┐
    │  │    CUT OUT 3D PATCH FROM VOLUME DATA │
    │  └─────────────────────────────────────┘
    │            │
    │            ▼                                    ⌐ S85
    │  ┌─────────────────────────────────────┐
    │  │ GENERATE MINIMUM INTENSITY PROJECTION│
    │  │          IMAGE OF EACH AXIS          │
    │  └─────────────────────────────────────┘
    │            │
    │            ▼                                    ⌐ S86
    │  ┌─────────────────────────────────────┐
    │  │       GENERATE COMBINED 2D PATCH     │
    │  └─────────────────────────────────────┘
    │            │
    │            ▼                                    ⌐ S87
    │  ┌─────────────────────────────────────┐
    │  │  MAKE INPUT TO CLASSIFIER TO PERFORM │
    │  │        IDENTIFICATION PROCESS        │
    │  └─────────────────────────────────────┘
    │            │
    │  NO        ▼                                    ⌐ S88
    └───────< HAVE ALL 3D PATCHES BEEN SELECTED? >
                 │ YES
                 ▼
              (  END  )
```

**REFERENCES CITED IN THE DESCRIPTION**

**Patent documents cited in the description**

- US 9968257 B
- US 20200320751

- US 20200380675 A1